# EUROPEAN PATENT APPLICATION

(11) **EP 4 556 000 A1**
(43) Date of publication of application: **21.05.2025**
(21) Application number: 23839684.0
(22) Date of filing: 13.07.2023
(51) Int. Cl.: A61K 9/19, A61K 31/7088, A61K 31/7105, A61K 31/713, A61K 47/22, A61K 48/00, A61P 35/00, A61P 43/00

(54) **LYOPHILIZED COMPOSITION OF NUCLEIC-ACID-LOADED LIPID NANOPARTICLES**

(30) Priority: 15.07.2022 JP 2022114394
(71) Applicant: NOF Corporation, Shibuya-ku Tokyo 150-6019 (JP); National University Corporation Chiba University, Chiba-shi, Chiba 263-8522 (JP)
(72) Inventor: TANGE, Kota, Kawasaki-shi, Kanagawa 210-0865 (JP); NAKAI, Yuta, Kawasaki-shi, Kanagawa 210-0865 (JP); TANEICHI, Sakura, Kawasaki-shi, Kanagawa 210-0865 (JP); KITAZAWA, Yui, Kawasaki-shi, Kanagawa 210-0865 (JP); AKITA, Hidetaka, Chiba-shi, Chiba 260-8675 (JP); TANAKA, Hiroki, Chiba-shi, Chiba 260-8675 (JP); SHIRANE, Daiki, Chiba-shi, Chiba 260-8675 (JP)
(74) Representative: Weickmann & Weickmann PartmbB
(86) International application number: PCT/JP2023/025892
(87) International publication number: WO 2024/014512

(57) **Abstract**

The present invention provides a lyophilized composition of nucleic acid-encapsulating lipid nanoparticles having a pH of 4.5 or more and 6.9 or less, including an ionic lipid represented by the formula (1): 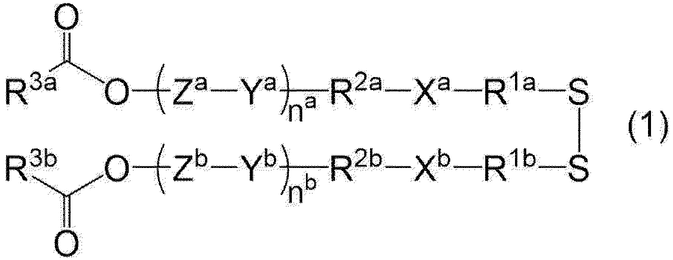 wherein symbols are as defined in the DESCRIPTION.

## Description

### [Technical Field]

The present invention relates to a lyophilized composition of nucleic acid-encapsulating lipid nanoparticles and a production method thereof, a method for producing nucleic acid-encapsulating lipid nanoparticles obtained by rehydrating a lyophilized composition, a method for producing a pharmaceutical composition containing a lyophilized composition or a nucleic acid-encapsulating lipid nanoparticle obtained by rehydrating a lyophilized composition, and a method for transferring nucleic acid into a cell or a target cell.

### [Background Art]

For practicalization of nucleic acid therapy using oligonucleic acids such as siRNA, and gene therapy using mRNA, pDNA, and the like, an effective and safe nucleic acid delivery carrier is demanded. While virus vectors are nucleic acid delivery carriers with good expression efficiency, the development of non-viral nucleic acid delivery carriers that can be used more safely is ongoing.

Since cationic liposomes using cationic lipids with quaternary amine are positively charged, they can form a complex (lipoplex) by electrostatic interaction with negatively-charged nucleic acids, and can deliver nucleic acids into cells. Utilizing the electrostatic interaction of quaternary amine with nucleic acid, it is also possible to prepare a lyophilized composition of cationic liposomes not containing nucleic acids and form a lipoplex by rehydration with an aqueous solution of nucleic acid. Thus, it has been shown that use thereof as a gene transfer reagent is available (see, for example, Patent Literatures 1 and 2).

However, it is difficult to control the particle size of lipoplex produced by such method, and cytotoxicity derived from positively-charged cationic lipids becomes a problem.

Therefore, lipid nanoparticles (LNP) using ionic lipids having a tertiary amine, which is positively charged under acidic conditions and has no electric charge under near neutral conditions, in the molecule were developed, and the lipid nanoparticles have become non-viral nucleic acid delivery carriers most generally used at present (see, for example, Non Patent Literature 1).

As examples of lipid nanoparticles using such ionic lipid having a tertiary amine in the molecule, those using ionic lipid having a degradable group are known (see, for example, Patent Literature 3).

As described above, various non-viral carriers have been developed. However, since nucleic acids are generally unstable compounds, there are still problems with their stability as pharmaceutical preparations.

### [Citation List]

### [Patent Literature]

[Patent Literature 1]
   JP 2008-5801 A
[Patent Literature 2]
   JP 2001-2565 A
[Patent Literature 3]
   WO 2013/073480
[Patent Literature 4]
   WO 2017/218704

### [Non Patent Literature]

[Non Patent Literature 1]
   Gene Therapy 6: 271-281, 1999
[Non Patent Literature 2]
   Biol. Pharm. Bull. 41, 1291-1294 (2018), Supplementary materials

### [Summary of Invention]

### [Technical Problem]

As one of the methods for improving the stability as a pharmaceutical preparation, attempts have been made to lyophilize lipid nanoparticles encapsulating nucleic acid and rehydrate them at the time of use to reconstitute the lipid nanoparticles (see, for example, Patent Literature 4 and Non Patent Literature 2). In this case, when lyophilizing nucleic acid-encapsulating lipid nanoparticles, the properties and gene delivery efficiency of the particles are required to stay unchanged even after a rehydration step following a lyophilizing step and storage.

For example, Non Patent Literature 2 discloses a method in which t-BuOH is used as a solvent for lipid, and lipid nanoparticles are prepared by mixing with a pH 4.0 malic acid buffer and lyophilized.

In addition, for example, Patent Literature 4 discloses a method in which lipid nanoparticles are prepared using EtOH as a solvent for lipid, which is exchanged with a pH 7.4 buffer, then an amphiphilic polymer is added, followed by lyophilization.

However, neither of the examples in Non Patent Literature 2 and Patent Literature 4 compares the gene delivery efficiency of lipid nanoparticles before lyophilization with that after storage and rehydration. That is, there was no technique to maintain the gene delivery efficiency before and after lyophilization. In addition, since the properties of lipid nanoparticles vary greatly depending on the ratio of ionic lipids and other components constituting the lipid nanoparticles, the lyophilization conditions need to be considered for each ionic lipid.

In view of the above-mentioned problems, the present invention aims to provide a lyophilized composition of nucleic acid-encapsulating lipid nanoparticles which has improved storage stability that could not be achieved with conventional techniques, and a production method thereof, a method for producing a pharmaceutical composition containing a lyophilized composition of nucleic acid-encapsulating lipid nanoparticles, and a method for transferring nucleic acid into cells or target cells.

### [Solution to Problem]

While lipid nanoparticles show strong interactions with nucleic acids under acidic conditions, they are preferably neutral from the aspect of toxicity when administered to cells or living organisms. The present inventors have conducted intensive studies in view of the above-mentioned problems and found that storage stability can be improved by preparing nucleic acid-encapsulating lipid nanoparticles in an acidic buffer, exchanging the buffer with another buffer having a buffering range of pH 4.5 to 6.9, and then lyophilizing same, which resulted in the completion of the present invention.

Accordingly, the present invention encompasses the following.
[1] A lyophilized composition of nucleic acid-encapsulating lipid nanoparticles having a pH of 4.5 or more and 6.9 or less, comprising an ionic lipid represented by the formula (1): 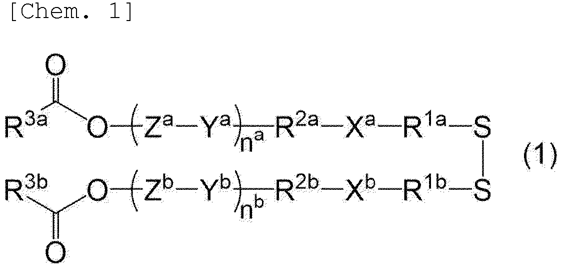
   (in the formula (1),
      R^{1a} and R^{1b} are each independently an alkylene group having 1 to 6 carbon atoms,
      X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
      R^{2a} and R^{2b} are each independently an alkylene group or an oxydialkylene group each having 8 or less carbon atoms,
      Y^{a} and Y^{b} are each independently an ester bond, an amide bond, a carbamate bond, an ether bond or a urea bond,
      Z^{a} and Z^{b} are each independently a divalent group derived from an aromatic compound having 3 to 16 carbon atoms and at least one aromatic ring, and optionally having a hetero atom,
      n^{a} and n^{b} are each independently 0 or 1, and
      R^{3a} and R^{3b} are each independently
      a residue derived from a reaction product of a liposoluble vitamin having a hydroxyl group, and succinic anhydride or glutaric anhydride,
      a residue derived from a reaction product of a sterol derivative having a hydroxyl group, and succinic anhydride or glutaric anhydride,
      an aliphatic hydrocarbon group having 1 to 40 carbon atoms,
      an alkyl group having 3 to 40 carbon atoms and a cyclopropane ring,
      a group represented by the formula (3):

         R⁹-O-CO-(CH₂)ₐ-* (3)
   (in the formula (3),
      * is a bonding position,
      R⁹ is an aliphatic hydrocarbon group having 2 to 20 carbon atoms, and
      a is an integer of 2 to 10),
      a group having 50 or less carbon atoms and represented by the formula (4): 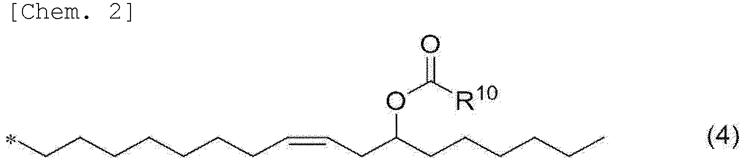
   (in the formula (4),
      * is a bonding position, and
      R¹⁰ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and R¹⁰ is optionally substituted by a substituent selected from the group consisting of a 3- to 14-membered heterocyclic group and a hydrocarbon ring group having 3 to 12 carbon atoms), or
      a group having 50 or less carbon atoms and represented by the formula (5): 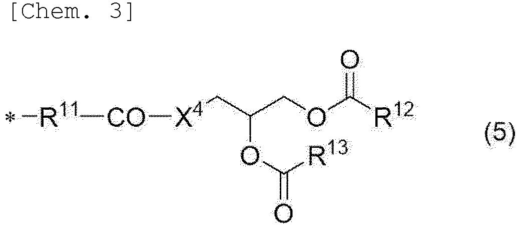
   (in the formula (5),
      * is a bonding position,
      R¹¹ is an alkylene group having 2 to 9 carbon atoms, an alkenediyl group having 2 to 9 carbon atoms, or an alkynediyl group having 2 to 9 carbon atoms, and R¹¹ is optionally substituted by a substituent selected from the group consisting of a halogen atom, a hydroxyl group, and a hydrocarbon ring group having 3 to 12 carbon atoms;
      R¹² and R¹³ are each independently an alkyl group having 1 to 17 carbon atoms, an alkenyl group having 2 to 17 carbon atoms, or an alkynyl group having 2 to 17 carbon atoms, and at least one ethylene group or at least one trimethylene group in R¹² is optionally replaced with at least one bond selected from the group consisting of an ester bond, an amide bond, a carbamate bond, and a carbonate bond, at least one ethylene group or at least one trimethylene group in R¹³ is optionally replaced with at least one bond selected from the group consisting of an ester bond, an amide bond, a carbamate bond, and a carbonate bond, and R¹² and R¹³ are each independently optionally substituted by a substituent selected from the group consisting of a halogen atom, a hydroxyl group, and a hydrocarbon ring group having 3 to 12 carbon atoms, and
      X⁴ is an oxygen atom, NH, or a sulfur atom).
[2] The lyophilized composition of the aforementioned [1], wherein
   R^{3a} and R^{3b} are each independently
   a residue derived from a reaction product of a liposoluble vitamin having a hydroxyl group, and succinic anhydride or glutaric anhydride,
   a residue derived from a reaction product of a sterol derivative having a hydroxyl group, and succinic anhydride or glutaric anhydride,
   an aliphatic hydrocarbon group having 1 to 40 carbon atoms,
   an alkyl group having 3 to 40 carbon atoms and a cyclopropane ring, or
   a group represented by the formula (3):

      R⁹-O-CO-(CH₂)ₐ-* (3)

      (the symbols in the formula (3) are defined as in [1]).
[3] The lyophilized composition of the aforementioned [1], wherein the aforementioned ionic lipid is a compound represented by the following formula: 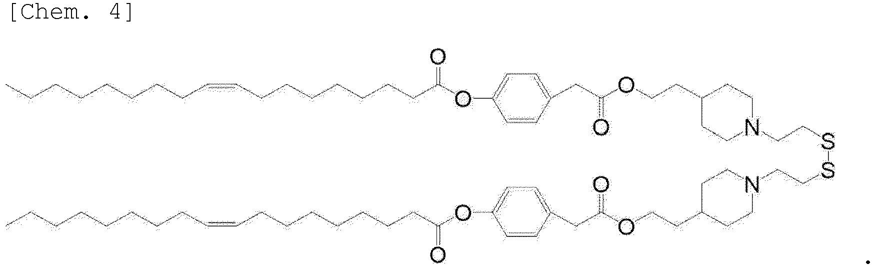
[4] The lyophilized composition of the aforementioned [3], further comprising a compound represented by the following formula: 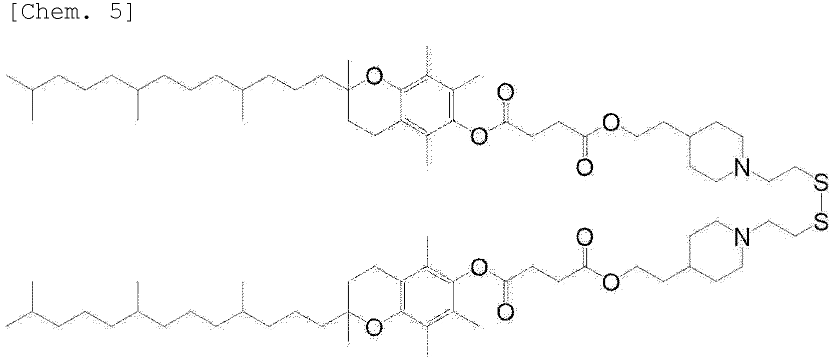 as the aforementioned ionic lipid.
[5] The lyophilized composition of the aforementioned [1], wherein the aforementioned ionic lipid is a compound represented by the following formula: 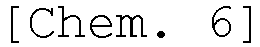 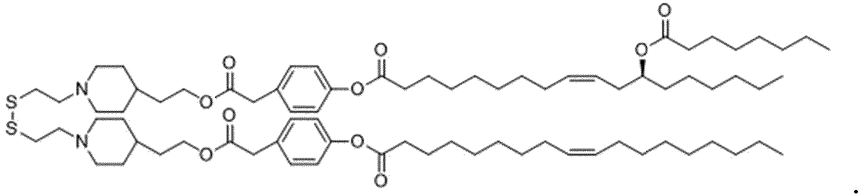
[6] The lyophilized composition of the aforementioned [1], wherein the aforementioned ionic lipid is a compound represented by the following formula: 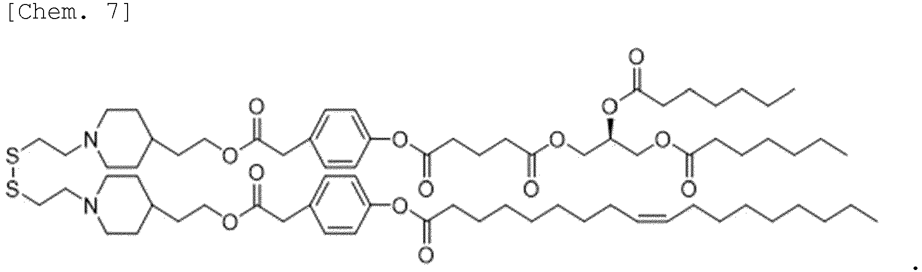
[7] The lyophilized composition of any one of the aforementioned [1] to [6], wherein the nucleic acid encapsulated in the aforementioned lipid nanoparticles is mRNA.
[8] A method for producing a lyophilized composition of nucleic acid-encapsulating lipid nanoparticles, comprising the following steps:
   a) mixing an alcohol solution comprising an ionic lipid represented by the aforementioned formula (1) in [1], a sterol, a phospholipid, and a PEG lipid with a nucleic acid solution in an acidic buffer having a buffering action at pH 1.0 to 6.5 to prepare a suspension of nucleic acid-encapsulating lipid nanoparticles;
   b) exchanging an external aqueous phase of the nucleic acid-encapsulating lipid nanoparticles with another buffer having a buffering action at pH 4.5 to 6.9 to obtain a mixture comprising the nucleic acid-encapsulating lipid nanoparticles at pH 4.5 to 6.9;
   c) mixing the obtained mixture with a cryoprotectant to obtain a mixture comprising 80 to 320 mg/mL of the cryoprotectant and the nucleic acid-encapsulating lipid nanoparticles at pH 4.5 to 6.9;
   d) lyophilizing the mixture obtained in step c) to obtain the lyophilized composition.
[9] A method for producing a nucleic acid-encapsulating lipid nanoparticle, comprising the following steps:
   a) mixing an alcohol solution comprising an ionic lipid represented by the aforementioned formula (1) in [1], a sterol, a phospholipid, and a PEG lipid with a nucleic acid solution in an acidic buffer having a buffering action at pH 1.0 to 6.5 to prepare a suspension of nucleic acid-encapsulating lipid nanoparticles;
   b) exchanging an external aqueous phase of the nucleic acid-encapsulating lipid nanoparticles with another buffer having a buffering action at pH 4.5 to 6.9 to obtain a mixture comprising the nucleic acid-encapsulating lipid nanoparticles at pH 4.5 to 6.9;
   c) mixing the obtained mixture with a cryoprotectant to obtain a mixture comprising 80 to 320 mg/mL of the cryoprotectant and the nucleic acid-encapsulating lipid nanoparticles at pH 4.5 to 6.9;
   d) lyophilizing the mixture obtained in step c) to obtain a lyophilized composition;
   e) mixing the lyophilized composition with water and optionally incubating the mixture at 0 to 95°C for 0 to 60 minutes to obtain the nucleic acid-encapsulating lipid nanoparticles.
[10] A method for transferring nucleic acid into cells, comprising a step of contacting the nucleic acid-encapsulating lipid nanoparticle produced by the method of the aforementioned [9] with cells in vitro.
[11] A method for transferring nucleic acid into target cells, comprising a step of administering the nucleic acid-encapsulating lipid nanoparticle produced by the method described in the aforementioned [9] to a living body.
[12] A method for producing a pharmaceutical composition, comprising the method of the aforementioned [8].
[13] A method for producing a pharmaceutical composition, comprising the method of the aforementioned [9].

### [Advantageous Effects of Invention]

The lyophilized composition of nucleic acid-encapsulating lipid nanoparticles of the present invention can be stored for a long period of time because the gene delivery efficiency decreases a little before and after lyophilization and the nucleic acid delivery efficiency does not change during storage. In addition, nucleic acid-encapsulating lipid nanoparticles prepared using the production method of the present invention show a small decrease in nucleic acid transfer efficiency before and after lyophilization compared to conventional techniques, and the nucleic acid delivery efficiency does not change during storage. Therefore, they are advantageous for gene transfer into cells and living organisms, and particularly useful as a pharmaceutical composition.

### [Brief Description of Drawings]

[Fig. 1]

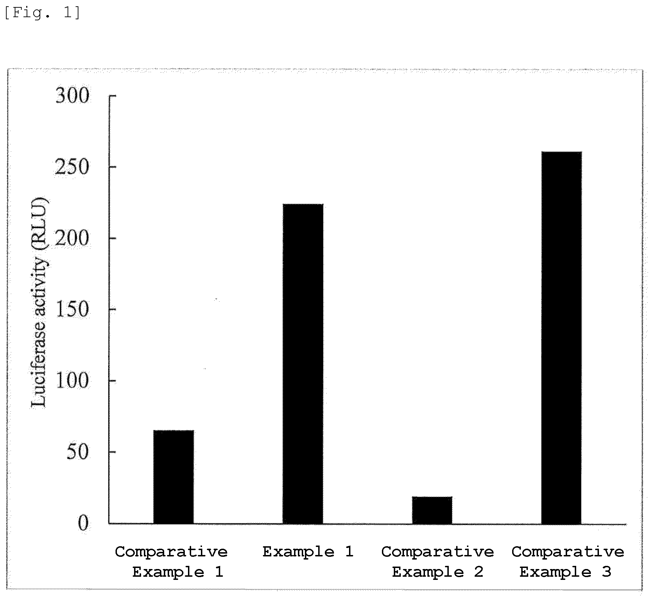

   Fig. 1 is a diagram evaluating the nucleic acid transfer efficiency of the nucleic acid-encapsulating lipid nanoparticles of Example 1, Comparative Example 1, Comparative Example 2, and Comparative Example 3.
[Fig. 2]

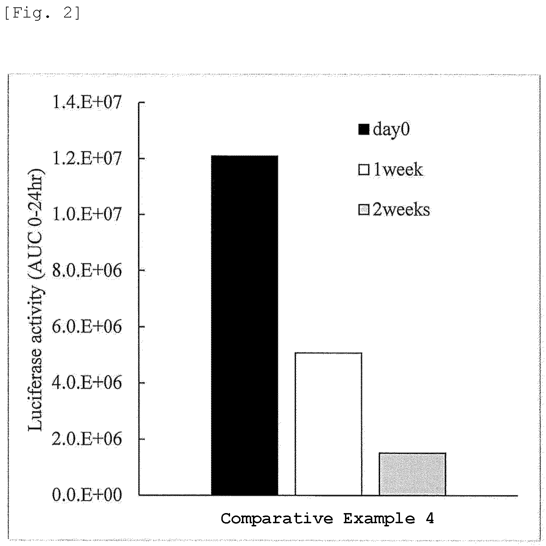

   Fig. 2 is a diagram evaluating changes over time in the nucleic acid transfer efficiency of the nucleic acid-encapsulating lipid nanoparticles of Comparative Example 4.
[Fig. 3]

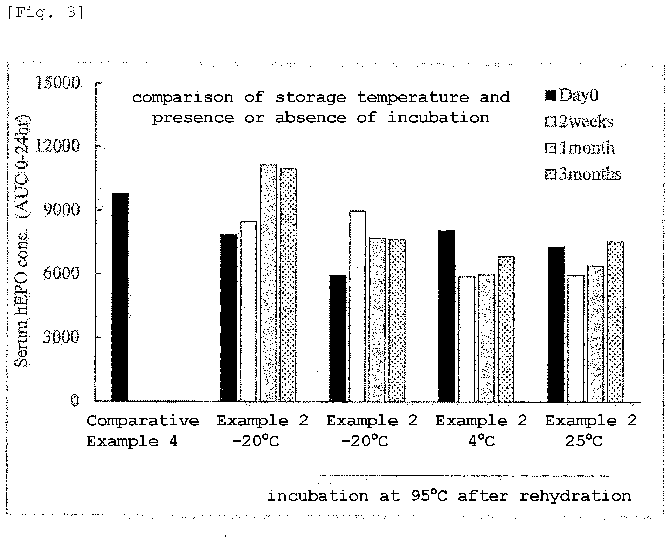

   Fig. 3 is a diagram evaluating influences of storage temperature and the presence or absence of incubation on the nucleic acid transfer efficiency.
[Fig. 4]

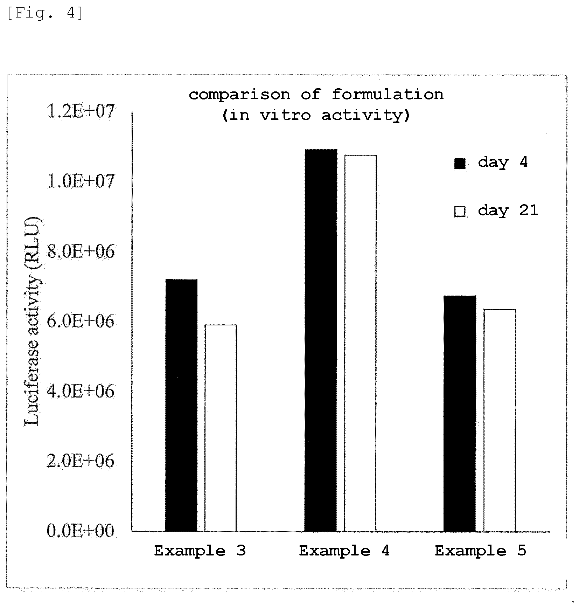

   Fig. 4 is a diagram evaluating influences of the formulation of ionic lipids for nucleic acid-encapsulating lipid nanoparticles, on the nucleic acid transfer efficiency.
[Fig. 5]

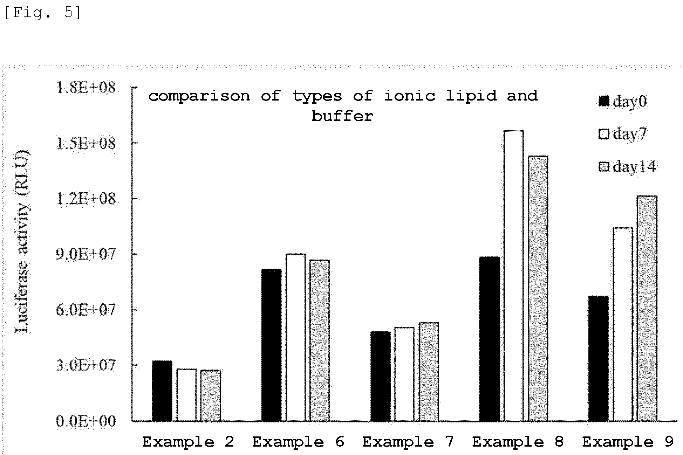

   Fig. 5 is a diagram evaluating influences of the type of ionic lipid and the type of buffer for nucleic acid-encapsulating lipid nanoparticles, on the nucleic acid transfer efficiency.

### [Description of Embodiments]

While the embodiments of the present invention are explained in the following, the present invention is not limited thereto.

The present invention provides a lyophilized composition of nucleic acid-encapsulating lipid nanoparticles having a pH of 4.5 or more and 6.9 or less.

The pH of a lyophilized composition can be measured by rehydrating the lyophilized composition. It does not change through a lyophilizing step or a rehydrating step. In the present invention, therefore, "a lyophilized composition of nucleic acid-encapsulating lipid nanoparticles having a pH of 4.5 or more and 6.9 or less" means that the pH measured after rehydration of the lyophilized composition is 4.5 or more and 6.9 or less, or the pH of the buffer solution used before lyophilizing is 4.5 or more and 6.9 or less. More specifically, "a lyophilized composition of nucleic acid-encapsulating lipid nanoparticles having a pH of 4.5 or more and 6.9 or less" means that the pH of the suspension of nucleic acid-encapsulating lipid nanoparticles obtained after rehydration of the lyophilized composition is 4.5 or more and 6.9 or less, or the pH of a buffer used as a dispersion medium of the suspension of nucleic acid-encapsulating lipid nanoparticles before lyophilizing is 4.5 or more and 6.9 or less (in other words, the pH of the suspension before lyophilizing in which the aforementioned nucleic acid-encapsulating lipid nanoparticles are dispersed in the aforementioned buffer is 4.5 or more and 6.9 or less). These pHs are preferably 5.0 or more and 6.9 or less, more preferably 5.5 or more and 6.9 or less.

A "lipid nanoparticle" means a particle having a membrane structure wherein the hydrophilic groups of amphiphilic lipid are arranged in the interface, facing the aqueous phase side. The "amphiphilic lipid" means a lipid having both a hydrophilic group showing hydrophilicity, and a hydrophobic group showing hydrophobicity. Examples of the amphiphilic lipid include ionic lipid, phospholipid, PEG lipid, and the like.

The lipid nanoparticles used in the present invention contain ionic lipid, a sterol, and PEG lipid as substances constituting a membrane, and may further contain a phospholipid. The particle size of the lipid nanoparticles is not particularly limited, and is preferably 10 nm to 500 nm, more preferably 30 nm to 300 nm. The particle size can be measured by using a particle size distribution measuring device such as Zetasizer Nano (Malvern) or the like. The particle size of the lipid nanoparticles can be appropriately adjusted by the method for producing the lipid nanoparticles. In the present invention, the "particle size" means an average particle size (number average) measured by a dynamic light scattering method.

In the present invention, the "total lipid" means the total amount of lipid. Examples of the lipid include ionic lipids, sterols, PEG lipids, and phospholipids.

In the present invention, the "nucleic acid-encapsulating lipid nanoparticle" means a lipid nanoparticle in which a nucleic acid is encapsulated inside the lipid nanoparticle.

### Ionic lipid

The ionic lipid that can be used in the present invention is a compound represented by the following formula (1), and a plurality of ionic lipids may be used in combination. 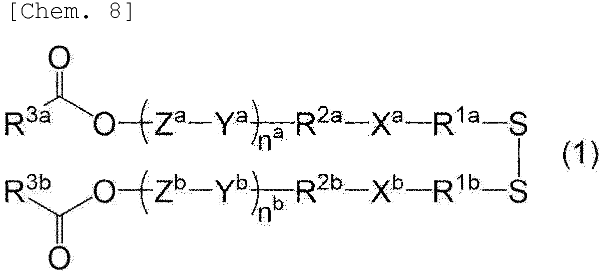
(in the formula (1),
   R^{1a} and R^{1b} are each independently an alkylene group having 1 to 6 carbon atoms,
   X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
   R^{2a} and R^{2b} are each independently an alkylene group or an oxydialkylene group each having 8 or less carbon atoms,
   Y^{a} and Y^{b} are each independently an ester bond, an amide bond, a carbamate bond, an ether bond or a urea bond,
   Z^{a} and Z^{b} are each independently a divalent group derived from an aromatic compound having 3 to 16 carbon atoms and at least one aromatic ring, and optionally having a hetero atom, and
   n^{a} and n^{b} are each independently 0 or 1,
   R^{3a} and R^{3b} are each independently
   a residue derived from a reaction product of a liposoluble vitamin having a hydroxyl group, and succinic anhydride or glutaric anhydride,
   a residue derived from a reaction product of a sterol derivative having a hydroxyl group, and succinic anhydride or glutaric anhydride,
   an aliphatic hydrocarbon group having 1 to 40 carbon atoms,
   an alkyl group having 3 to 40 carbon atoms and a cyclopropane ring,
   a group represented by the formula (3):

      R⁹-O-CO-(CH₂)ₐ-* (3)
(in the formula (3),
   * is a bonding position,
   R⁹ is an aliphatic hydrocarbon group having 2 to 20 carbon atoms, and
   a is an integer of 2 to 10),
   a group having 50 or less carbon atoms and represented by the formula (4): 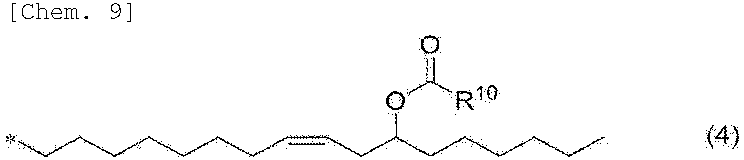
(in the formula (4),
   * is a bonding position, and
   R¹⁰ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and R¹⁰ is optionally substituted by a substituent selected from the group consisting of a 3- to 14-membered heterocyclic group and a hydrocarbon ring group having 3 to 12 carbon atoms), or
   a group having 50 or less carbon atoms and represented by the formula (5): 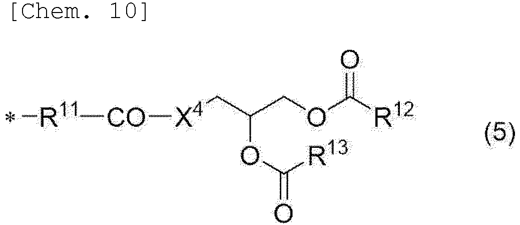
(in the formula (5),
   * is a bonding position,
   R¹¹ is an alkylene group having 2 to 9 carbon atoms, an alkenediyl group having 2 to 9 carbon atoms, or an alkynediyl group having 2 to 9 carbon atoms, and R¹¹ may be substituted by a substituent selected from the group consisting of a halogen atom, a hydroxyl group, and a hydrocarbon ring group having 3 to 12 carbon atoms,
   R¹² and R¹³ are each independently an alkyl group having 1 to 17 carbon atoms, an alkenyl group having 2 to 17 carbon atoms, or an alkynyl group having 2 to 17 carbon atoms, and at least one ethylene group or at least one trimethylene group in R¹² is optionally replaced with at least one bond selected from the group consisting of an ester bond, an amide bond, a carbamate bond, and a carbonate bond, at least one ethylene group or at least one trimethylene group in R¹³ is optionally replaced with at least one bond selected from the group consisting of an ester bond, an amide bond, a carbamate bond, and a carbonate bond, and R¹² and R¹³ are each independently optionally substituted by a substituent selected from the group consisting of a halogen atom, a hydroxyl group, and a hydrocarbon ring group having 3 to 12 carbon atoms, and
   X⁴ is an oxygen atom, NH, or a sulfur atom).

The definition of each symbol in the formula (1) is explained below in order.
R^{1a} and R^{1b} are each independently an alkylene group having 1 to 6 carbon atoms, and may be linear or branched, preferably linear. The carbon number of the alkylene group is preferably 1 to 4, more preferably 1 to 2. Specific examples of the alkylene group having 1 to 6 carbon atoms include methylene group, ethylene group, trimethylene group, isopropylene group, tetramethylene group, isobutylene group, pentamethylene group, neopentylene group, and the like. Preferably, R^{1a} and R^{1b} are each independently a methylene group, an ethylene group, a trimethylene group, an isopropylene group or a tetramethylene group, most preferably an ethylene group.
R^{1a} may be the same as or different from R^{1b}, and R^{1a} is preferably the same group as R^{1b}.
X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups, preferably each independently a cyclic alkylene tertiary amino group having 2 to 5 carbon atoms and 1 to 2 tertiary amino groups.

The alkyl group having 1 to 6 carbon atoms in the acyclic alkyl tertiary amino group having 1 to 6 carbon atoms and one tertiary amino group may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group, and the like. It is preferably a methyl group, an ethyl group, a propyl group, or an isopropyl group, most preferably a methyl group.

A preferred specific structure of the acyclic alkyl tertiary amino group having 1 to 6 carbon atoms and one tertiary amino group is represented by X¹. The both ends of the following formula for X¹ are not carbon atoms but the bonding positions.

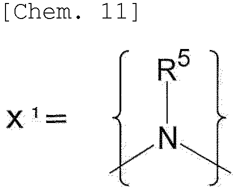

R⁵ in X¹ is an alkyl group having 1 to 6 carbon atoms, which may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group, and the like. It is preferably a methyl group, an ethyl group, a propyl group, or an isopropyl group, most preferably a methyl group.

The carbon number of the cyclic alkylene tertiary amino group having 2 to 5 carbon atoms and 1 to 2 tertiary amino groups is preferably 4 to 5. The cyclic alkylene tertiary amino group having 2 to 5 carbon atoms and 1 to 2 tertiary amino groups is specifically an aziridylene group, an azetidylene group, a pyrrolidylene group, a piperidylene group, an imidazolidylene group, or a piperazylene group, preferably a pyrrolidylene group, a piperidylene group, or a piperazylene group, most preferably a piperidylene group.

A preferred specific structure of the cyclic alkylene tertiary amino group having 2 to 5 carbon atoms and one tertiary amino group is represented by X². The both ends of the following formula for X² are not carbon atoms but the bonding positions.

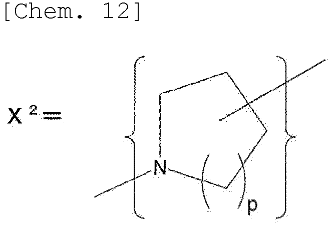

The p in X² is 1 or 2. When p is 1, X² is a pyrrolidylene group, and when p is 2, X² is a piperidylene group. Preferably, p is 2.

A preferred specific structure of the cyclic alkylene tertiary amino group having 2 to 5 carbon atoms and two tertiary amino groups is represented by X³. The both ends of the following formula for X³ are not carbon atoms but the bonding positions.

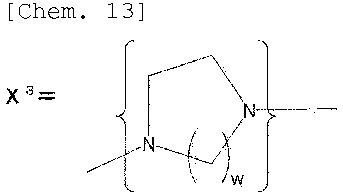

The w in X³ is 1 or 2. When w is 1, X³ is an imidazolidylene group, and when w is 2, X³ is a piperazylene group.

X² may be the same as or different from X^{b}, and X^{a} is preferably the same group as X^{b}.

R^{2a} and R^{2b} are each independently an alkylene group or an oxydialkylene group each having 8 or less carbon atoms, preferably each independently an alkylene group having 8 or less carbon atoms.

The alkylene group having 8 or less carbon atoms may be linear or branched, preferably linear. The number of carbons contained in the alkylene group is preferably 6 or less, most preferably 4 or less. Specific examples of the alkylene group having 8 or less carbon atoms include methylene group, ethylene group, trimethylene group, isopropylene group, tetramethylene group, isobutylene group, pentamethylene group, hexamethylene group, heptamethylene group, octamethylene group, and the like. It is preferably a methylene group, an ethylene group, a trimethylene group, or a tetramethylene group, most preferably an ethylene group.

In the present specification, the "oxydialkylene group having 8 or less carbon atoms" is alkylene groups via an ether bond (alkylene-O-alkylene, "alkyleneoxyalkylene group"), wherein the total carbon number of the two alkylene groups present is 8 or below. The two alkylene groups present may be the same or different, preferably the same. Specific examples of the oxydialkylene group having 8 or less carbon atoms include oxydimethylene group, oxydiethylene group, oxydi(trimethylene) group (i.e., trimethyleneoxytrimethylene group), oxydi(tetramethylene) group (i.e., tetramethyleneoxytetramethylene group), and the like. It is preferably an oxydimethylene group, an oxydiethylene group, or an oxydi(trimethylene) group, most preferably an oxydiethylene group.

R^{2a} may be the same as or different from R^{2b}, and R^{2a} is preferably the same group as R^{2b}.

Y^{a} and Y^{b} are each independently an ester bond, an amide bond, a carbamate bond, an ether bond or a urea bond, preferably each independently an ester bond, an amide bond or a carbamate bond, more preferably each independently an ester bond or an amide bond, most preferably each an ester bond. The direction of the bond of Y^{a} and Y^{b} is not limited. When Y^{a} and Y^{b} are ester bonds, the structure of -Z^{a}-CO-O-R^{2a}- or -Z^{b}-CO-OR^{2b}- is preferably shown.

Y^{a} may be the same as or different from Y^{b}, and Y^{a} is preferably the same group as Y^{b}.

Z^{a} and Z^{b} are each independently a divalent group derived from an aromatic compound having 3 to 16 carbon atoms and at least one aromatic ring, and optionally having a hetero atom. The number of carbons contained in the aromatic compound is preferably 6 to 12, most preferably 6 to 7. The aromatic ring contained in the aromatic compound is preferably one.

As the kind of the aromatic ring contained in the aromatic compound having 3 to 16 carbon atoms, benzene ring, naphthalene ring, and anthracene ring can be mentioned for aromatic hydrocarbon ring, and imidazole ring, pyrazole ring, oxazole ring, isoxazole ring, thiazole ring, isothiazole ring, triazine ring, pyrrole ring, furanthiophene ring, pyrimidine ring, pyridazine ring, pyrazine ring, pyridine ring, purine ring, pteridine ring, benzimidazole ring, indole ring, benzofuran ring, quinazoline ring, phthalazine ring, quinoline ring, isoquinoline ring, coumarin ring, chromone ring, benzodiazepine ring, phenoxazine ring, phenothiazine ring, acridine ring, and the like can be mentioned for aromatic heterocycle. It is preferably a benzene ring, a naphthalene ring, or an anthracene ring, most preferably a benzene ring.

The aromatic ring may have a substituent. Examples of the substituent include acyl group having 2 to 4 carbon atoms, alkoxycarbonyl group having 2 to 4 carbon atoms, carbamoyl group having 2 to 4 carbon atoms, acyloxy group having 2 to 4 carbon atoms, acylamino group having 2 to 4 carbon atoms, alkoxycarbonylamino group having 2 to 4 carbon atoms, fluorine atom, chlorine atom, bromine atom, iodine atom, alkylsulfanyl group having 1 to 4 carbon atoms, alkylsulfonyl group having 1 to 4 carbon atoms, arylsulfonyl group having 6 to 10 carbon atoms, nitro group, trifluoromethyl group, cyano group, alkyl group having 1 to 4 carbon atoms, ureido group having 1 to 4 carbon atoms, alkoxy group having 1 to 4 carbon atoms, aryl group having 6 to 10 carbon atoms, aryloxy group having 6 to 10 carbon atoms, and the like. Preferred examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group, and the like.

A preferred specific structure of Z^{a} and Z^{b} is Z¹. The both ends of the following formula for Z¹ are not carbon atoms but the bonding positions.

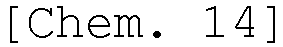

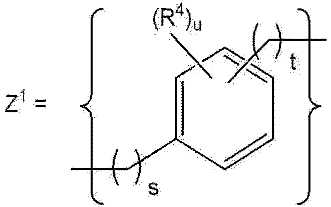

wherein s is an integer of 0 to 3, t is an integer of 0 to 3, u is an integer of 0 to 4, and R⁴ in the number of u are each independently a substituent.

The s in Z¹ is preferably an integer of 0 to 1, more preferably 0.

The t in Z¹ is preferably an integer of 0 to 2, more preferably 1.

The u in Z¹ is preferably an integer of 0 to 2, more preferably an integer of 0 to 1.

The R⁴ in Z¹ is a substituent of an aromatic ring (benzene ring) contained in the aromatic compound having 3 to 16 carbon atoms which does not inhibit the reaction in the synthesis process of an ionic lipid. Examples of the substituent include acyl group having 2 to 4 carbon atoms, alkoxycarbonyl group having 2 to 4 carbon atoms, carbamoyl group having 2 to 4 carbon atoms, acyloxy group having 2 to 4 carbon atoms, acylamino group having 2 to 4 carbon atoms, alkoxycarbonylamino group having 2 to 4 carbon atoms, fluorine atom, chlorine atom, bromine atom, iodine atom, alkylsulfanyl group having 1 to 4 carbon atoms, alkylsulfonyl group having 1 to 4 carbon atoms, arylsulfonyl group having 6 to 10 carbon atoms, nitro group, trifluoromethyl group, cyano group, alkyl group having 1 to 4 carbon atoms, ureido group having 1 to 4 carbon atoms, alkoxy group having 1 to 4 carbon atoms, aryl group having 6 to 10 carbon atoms, aryloxy group having 6 to 10 carbon atoms, and the like. Preferred examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group, and the like. When R⁴ is present in plurality, each R⁴ may be the same or different.

Z^{a} may be the same as or different from Z^{b}, and Z^{a} is preferably the same group as Z^{b}.

n^{a} and n^{b} are each independently 0 or 1.

n^{a} may be the same as or different from n^{b}, and n^{a} is preferably the same group as n^{b}.

R^{3a} and R^{3b} are each independently a residue derived from a reaction product of a liposoluble vitamin having a hydroxyl group, and succinic anhydride or glutaric anhydride, or a residue derived from a reaction product of a sterol derivative having a hydroxyl group, and succinic anhydride or glutaric anhydride, an aliphatic hydrocarbon group having 1 to 40 carbon atoms, an alkyl group having 3 to 40 carbon atoms and a cyclopropane ring, a group represented by the aforementioned formula (3), a group having 50 or less carbon atoms and represented by the aforementioned formula (4), or a group having 50 or less carbon atoms and represented by the aforementioned formula (5).

In one embodiment of the present invention, R^{3a} and R^{3b} are preferably each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 to 40 carbon atoms, an alkyl group having 3 to 40 carbon atoms and a cyclopropane ring, or a group represented by the aforementioned formula (3),
more preferably each independently a residue derived from a reaction product of a liposoluble vitamin having a hydroxyl group, and succinic anhydride or glutaric anhydride, or an aliphatic hydrocarbon group having 12 to 22 carbon atoms,
further preferably each independently an aliphatic hydrocarbon group having 12 to 22 carbon atoms.

In another embodiment of the present invention, R^{3a} and R^{3b} are preferably each independently a residue derived from a reaction product of a liposoluble vitamin having a hydroxyl group, and succinic anhydride or glutaric anhydride, an aliphatic hydrocarbon group having 12 to 22 carbon atoms, a group having 50 or less carbon atoms and represented by the aforementioned formula (4), or a group having 50 or less carbon atoms and represented by the aforementioned formula (5).

In another embodiment of the present invention, more preferably,
R^{3a} and R^{3b} are each independently a residue derived from a reaction product of a liposoluble vitamin having a hydroxyl group, and succinic anhydride or glutaric anhydride,
R^{3a} and R^{3b} are each independently an aliphatic hydrocarbon group having 12 to 22 carbon atoms,
one of R^{3a} and R^{3b} is an aliphatic hydrocarbon group having 12 to 22 carbon atoms and the remaining one is a group having 50 or less carbon atoms and represented by the aforementioned formula (4), or
one of R^{3a} and R^{3b} is an aliphatic hydrocarbon group having 12 to 22 carbon atoms and the remaining one is a group having 50 or less carbon atoms and represented by the aforementioned formula (5).

In another embodiment of the present invention, further preferably,
R^{3a} and R^{3b} are each independently a residue derived from a reaction product of a liposoluble vitamin having a hydroxyl group, and succinic anhydride or glutaric anhydride, or
R^{3a} and R^{3b} are each independently an aliphatic hydrocarbon group having 12 to 22 carbon atoms.

The "residue derived from a reaction product of a liposoluble vitamin having a hydroxyl group, and succinic anhydride or glutaric anhydride" is a group having a structure in which the hydroxyl group of the liposoluble vitamin having the hydroxyl group is replaced with *-O-CO-CH₂-CH₂- or *-O-CO-CH₂-CH₂-CH₂-. * shows the bonding position with the liposoluble vitamin.

The "residue derived from a reaction product of a sterol derivative having a hydroxyl group, and succinic anhydride or glutaric anhydride" is a group having a structure in which the hydroxyl group of the sterol derivative having the hydroxyl group is replaced with *-O-CO-CH₂-CH₂- or *-O-CO-CH₂-CH₂-CH₂-. * shows the bonding position with the sterol derivative.

Examples of the liposoluble vitamin having a hydroxyl group include retinol, ergosterol, 7-dehydrocholesterol, calciferol, cholecalciferol, dihydroergocalciferol, dihydrotachysterol, tocopherol, tocotrienol, and the like. The liposoluble vitamin having a hydroxyl group is preferably tocopherol.

Examples of the sterol derivative having a hydroxyl group include cholesterol, cholestanol, stigmasterol, β-sitosterol, lanosterol, ergosterol, and the like. It is preferably cholesterol or cholestanol.

The aliphatic hydrocarbon group having 1 to 40 carbon atoms may be linear or branched. The aliphatic hydrocarbon group may be saturated or unsaturated. In the case of an unsaturated aliphatic hydrocarbon group, the aliphatic hydrocarbon group generally contains 1 to 6, preferably 1 to 3, more preferably 1 to 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 12 to 22, more preferably 13 to 19, most preferably 13 to 17. While the aliphatic hydrocarbon group includes an alkyl group, an alkenyl group, an alkynyl group, and the like, it is preferably an alkyl group or an alkenyl group. Specific examples of the aliphatic hydrocarbon group having 1 to 40 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, henicosyl group, docosyl group, tricosyl group, tetracosyl group, pentacosyl group, hexacosyl group, heptacosyl group, octacosyl group, nonacosyl group, triacontyl group, tetracontyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, decadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, docosadienyl group, octadecatrienyl group, icosatrienyl group, icosatetraenyl group, icosapentaenyl group, docosahexaenyl group, isostearyl group, 1-hexylheptyl group, 1-hexylnonyl group, 1-octylnonyl group, 1-octylundecyl group, 1-decylundecyl group, and the like. The aliphatic hydrocarbon group having 1 to 40 carbon atoms is preferably a tridecyl group, a pentadecyl group, a heptadecyl group, a nonadecyl group, a heptadecenyl group, a heptadecadienyl group, or a 1-hexylnonyl group, particularly preferably a tridecyl group, a heptadecyl group, a heptadecenyl group, or a heptadecadienyl group.

In one embodiment of the present invention, the aliphatic hydrocarbon group having 1 to 40 (preferably 12 to 22) carbon atoms for R^{3a} or R^{3b} is derived from fatty acid. In this case, the carbonyl carbon derived from fatty acid is contained in - CO-O- in the formula (1). A specific example of the aliphatic hydrocarbon group is a heptadecadienyl group when linoleic acid is used as the fatty acid, or a heptadecenyl group when oleic acid is used as the fatty acid.

The "alkyl group having 3 to 40 carbon atoms and a cyclopropane ring" for R^{3a} or R^{3b} mean an alkyl group having 3 to 40 carbon atoms having at least one cyclopropane ring in the alkyl chain. The carbon number 3 to 40 in the alkyl group does not include the number of carbon atoms in the cyclopropane ring. The number of cyclopropane rings in the alkyl group is preferably one. The alkyl group having 3 to 40 carbon atoms and a cyclopropane ring for R^{3a} or R^{3b} is preferably a group represented by the formula (6): 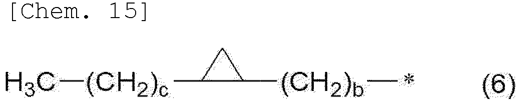 (in the formula (6), * is a bonding position, b and c are each independently an integer and the total of b and c is 2 to 39). Preferably, b is an integer of 1 to 20 and c is an integer of 1 to 19. b is more preferably an integer of 2 to 18, further preferably an integer of 3 to 17, further more preferably an integer of 4 to 12. c is more preferably an integer of 3 to 15, further preferably an integer of 3 to 11, further more preferably an integer of 3 to 9. Examples of the group represented by the formula (6) include 7-(2-octylcyclopropyl)heptyl and the like.

In the formula (3), * is not carbon atom but the bonding position, as mentioned above.

The aliphatic hydrocarbon group having 2 to 20 carbon atoms, which is represented by R⁹ in the formula (3) may be linear or branched.

The aforementioned aliphatic hydrocarbon group may be saturated or unsaturated. When the aforementioned aliphatic hydrocarbon group is an unsaturated aliphatic hydrocarbon group, the aliphatic hydrocarbon group generally contains 1 to 6, preferably 1 to 3, more preferably 1 to 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 8 to 20, more preferably 9 to 19, further preferably 13 to 19, most preferably 13 to 17. While the aliphatic hydrocarbon group includes an alkyl group, an alkenyl group, an alkynyl group, and the like, it is preferably an alkyl group or an alkenyl group, more preferably an alkyl group. Specific examples of the aliphatic hydrocarbon group having 2 to 20 carbon atoms include ethyl group, propyl group, isopropyl group, n-butyl group, sec-butyl group, isobutyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, icosatrienyl group, icosatetraenyl group, icosapentaenyl group, isostearyl group, 1-hexylheptyl group, 1-ethylnonyl group, 1-butylnonyl group, 1-hexylnonyl group, 1-octylnonyl group, 1-octylundecyl group, 3-octylundecyl group, and the like. The aliphatic hydrocarbon group having 2 to 20 carbon atoms is preferably a tridecyl group, a pentadecyl group, a heptadecyl group, a nonadecyl group, a heptadecenyl group, a heptadecadienyl group, or a 1-hexylnonyl group, particularly preferably a tridecyl group, a heptadecyl group, a heptadecenyl group, or a heptadecadienyl group.

In the formula (3), a is preferably an integer of 3 to 9, more preferably an integer of 3 to 7, further preferably an integer of 5 to 7, most preferably 5 or 7.

In the formula (4), * is not carbon atom but the bonding position, as mentioned above.

In the formula (4), R¹⁰ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and R¹⁰ is optionally substituted by a substituent selected from the group consisting of a 3- to 14-membered heterocyclic group and a hydrocarbon ring group having 3 to 12 carbon atoms.

In the present specification, "R¹⁰ is optionally substituted by a substituent selected from the group consisting of a 3- to 14-membered heterocyclic group and a hydrocarbon ring group having 3 to 12 carbon atoms" (hereinafter abbreviated as "the aforementioned expression") means that the aforementioned alkyl group, the aforementioned alkenyl group, the aforementioned alkynyl group, or the aforementioned hydrocarbon ring group for R¹⁰ may each be independently substituted by a substituent selected from the group consisting of a 3- to 14-membered heterocyclic group and a hydrocarbon ring group having 3 to 12 carbon atoms. Other expressions similar to the aforementioned expression have the same meaning as the aforementioned expression. In the present specification, unless otherwise specified regarding the substituent, "alkyl group", "alkenyl group", "alkynyl group", "alkylene group", "alkenediyl group", and "alkynediyl group" refer to those not substituted.

In the present specification, the alkyl group may be either linear or branched. Examples of the alkyl group include methyl group, ethyl group, propyl group, isopropyl group, butyl group, isobutyl group, sec-butyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, henicosyl group, docosyl group, tricosyl group, tetracosyl group, pentacosyl group, hexacosyl group, heptacosyl group, octacosyl group, nonacosyl group, triacontyl group, hentriacontyl group, dotriacontyl group, tritriacontyl group, tetratriacontyl group, pentatriacontyl group, hexatriacontyl group, tetracontyl group, hentetracontyl group, dotetracontyl group, tritetracontyl group, and tetratetracontyl group.

In the present specification, the alkenyl group may be either linear or branched. In the present specification, the number of olefinic carbon-carbon double bonds in the alkenyl group may be only one, or two or more. Examples of the alkenyl group include ethenyl group, propenyl group, butenyl group, pentenyl group, hexenyl group, heptenyl group, octenyl group, nonenyl group, decenyl group, undecenyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, tricosenyl group, tetracosenyl group, pentacosenyl group, hexacosenyl group, heptacosenyl group, octacosenyl group, nonacosenyl group, triacontenyl group, hentriacontenyl group, and dotriacontenyl group.

In the present specification, the alkynyl group may be either linear or branched. In the present specification, the number of carbon-carbon triple bonds in the alkynyl group may be only one, or two or more. Examples of the alkynyl group include ethynyl group, propynyl group, butynyl group, pentynyl group, hexynyl group, heptynyl group, octynyl group, nonynyl group, decynyl group, undecynyl group, dodecynyl group, tridecynyl group, tetradecynyl group, pentadecynyl group, hexadecynyl group, heptadecynyl group, octadecynyl group, nonadecynyl group, icosynyl group, henicosynyl group, docosynyl group, tricosynyl group, tetracosynyl group, pentacosynyl group, hexacosynyl group, heptacosynyl group, octacosynyl group, nonacosynyl group, triacontinyl group, hentriacontinyl group, and dotriacontinyl group.

In the present specification, "a hydrocarbon ring group having 3 to 12 carbon atoms" refers to a cyclic group having a ring composed of 3 to 12 carbon atoms. Examples of the hydrocarbon ring group having 3 to 12 carbon atoms include cycloalkyl group having 3 to 8 carbon atoms, phenyl group, naphthyl group, and adamantyl group. Examples of the cycloalkyl group having 3 to 8 carbon atoms include cyclopropyl group, cyclobutyl group, cyclopentyl group, cyclohexyl group, cycloheptyl group, and cyclooctyl group. The hydrocarbon ring group having 3 to 12 carbon atoms is preferably a non-aromatic hydrocarbon ring group having 3 to 12 carbon atoms, more preferably a cycloalkyl group having 3 to 8 carbon atoms or adamantyl group, further preferably a cyclohexyl group or an adamantyl group.

In the present specification, the "3- to 14-membered heterocyclic group" refers to a heterocyclic group having 3 to 14 ring-constituting atoms. Examples of the 3- to 14-membered heterocyclic group include a 5- to 14-membered aromatic heterocyclic group and a 3- to 14-membered non-aromatic heterocyclic group.

In the present specification, examples of the 5- to 14-membered aromatic heterocyclic group include the following:
(i) 5- or 6-membered monocyclic aromatic heterocyclic groups such as thienyl group, furyl group, pyrrolyl group, imidazolyl group, pyrazolyl group, thiazolyl group, isothiazolyl group, oxazolyl group, isoxazolyl group, pyridyl group, pyrazinyl group, pyrimidinyl group, pyridazinyl group, 1,2,4-oxadiazolyl group, 1,3,4-oxadiazolyl group, 1,2,4-thiadiazolyl group, 1,3,4-thiadiazolyl group, triazolyl group, tetrazolyl group, triazinyl group, and the like;
(ii) 8- to 14-membered fused polycyclic aromatic heterocyclic groups such as benzothiophenyl group, benzofuranyl group, benzoimidazolyl group, benzoxazolyl group, benzoisoxazolyl group, benzothiazolyl group, benzoisothiazolyl group, benzotriazolyl group, imidazopyridinyl group, thienopyridinyl group, furopyridinyl group, pyrrolopyridinyl group, pyrazolopyridinyl group, oxazolopyridinyl group, thiazolopyridinyl group, imidazopyrazinyl group, imidazopyrimidinyl group, thienopyrimidinyl group, furopyrimidinyl group, pyrrolopyrimidinyl group, pyrazolopyrimidinyl group, oxazolopyrimidinyl group, thiazolopyrimidinyl group, pyrazolotriazinyl group, naphtho[2,3-b]thienyl group, phenoxathiinyl group, indolyl group, isoindolyl group, 1H-indazolyl group, purinyl group, isoquinolyl group, quinolyl group, phthalazinyl group, naphthyridinyl group, quinoxalinyl group, quinazolinyl group, cinnolinyl group, carbazolyl group, β-carbolinyl group, phenanthridinyl group, acrydinyl group, phenazinyl group, phenothiazinyl group, phenoxazinyl group, and the like.

In the present specification, examples of the 3- to 14-membered non-aromatic heterocyclic group include the following:
(i) 3- to 8-membered monocyclic non-aromatic heterocyclic groups such as aziridinyl group, oxiranyl group, thiiranyl group, azetidinyl group, oxetanyl group, thietanyl group, tetrahydrothienyl group, tetrahydrofuranyl group, pyrrolinyl group, pyrrolidinyl group, imidazolinyl group, imidazolidinyl group, oxazolinyl group, oxazolidinyl group, pyrazolinyl group, pyrazolidinyl group, thiazolinyl group, thiazolidinyl group, tetrahydroisothiazolyl group, tetrahydrooxazolyl group, tetrahydroisoxazolyl group, piperidinyl group, piperazinyl group, tetrahydropyridinyl group, dihydropyridinyl group, dihydrothiopyranyl group, tetrahydropyrimidinyl group, tetrahydropyridazinyl group, dihydropyranyl group, tetrahydropyranyl group, tetrahydrothiopyranyl group, morpholinyl group, thiomorpholinyl group, azepanyl group, diazepanyl group, azepinyl group, oxepanyl group, azocanyl group, diazocanyl group, and the like;
(ii) 9- to 14-membered fused polycyclic non-aromatic heterocyclic groups such as dihydrobenzofuranyl group, dihydrobenzoimidazolyl group, dihydrobenzoxazolyl group, dihydrobenzothiazolyl group, dihydrobenzoisothiazolyl group, dihydronaphto[2,3-b]thienyl group, tetrahydroisoquinolyl group, tetrahydroquinolyl group, 4H-quinolizinyl group, indolinyl group, isoindolinyl group, tetrahydrothieno[2,3-c]pyridinyl group, tetrahydrobenzoazepinyl group, tetrahydroquinoxalinyl group, tetrahydrophenanthridinyl group, hexahydrophenothiazinyl group, hexahydrophenoxazinyl group, tetrahydrophthalazinyl group, tetrahydronaphthyridinyl group, tetrahydroquinazolinyl group, tetrahydrocinnolinyl group, tetrahydrocarbazolyl group, tetrahydro-β-carbolinyl group, tetrahydroacrydinyl group, tetrahydrophenazinyl group, tetrahydrothioxanthenyl group, octahydroisoquinolyl group, and the like.

R¹⁰ is preferably an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and the aforementioned alkyl group is optionally substituted by a hydrocarbon ring group having 3 to 12 carbon atoms.

R¹⁰ is more preferably an alkyl group having 1 to 20 carbon atoms, an alkenyl group having 2 to 20 carbon atoms, an alkynyl group having 2 to 20 carbon atoms, or a cyclohexyl group, and the aforementioned alkyl group is optionally substituted by an adamantyl group.

R¹⁰ is further preferably an alkyl group having 1 to 20 carbon atoms, particularly preferably an alkyl group having 1 to 10 carbon atoms, most preferably a heptyl group.

In the formula (5), * is not carbon atom but the bonding position, as described above.

In the formula (5), R¹¹ is an alkylene group having 2 to 9 carbon atoms, an alkenediyl group having 2 to 9 carbon atoms, or an alkynediyl group having 2 to 9 carbon atoms, and R¹¹ is optionally substituted by a substituent selected from the group consisting of a halogen atom, a hydroxyl group, and a hydrocarbon ring group having 3 to 12 carbon atoms.

In the present specification, the alkylene group may be **either** linear or branched. Examples of the alkylene group include methylene group, ethylene group, trimethylene group (-(CH₂)₃-), propylene group (-CH(CH₃)CH₂-, -CH₂CH(CH₃) -), tetramethylene group (- (CH₂)₄-), butylene group (-CH (C₂H₅)CH₂-, -CH₂CH(C₂H₅)-), pentamethylene group (- (CH₂)₅-), hexamethylene group (-(CH₂)₆-), heptamethylene group (- (CH₂)₇-), octamethylene group (-(CH₂)₈-), nonamethylene group (-(CH₂)₉-), and decamethylene group (-(CH₂)₉-) (in the aforementioned formula, **"-"** is a single bond).

In the present specification, the "alkenediyl group" refers to a divalent group having a structure obtained by removing two hydrogen atoms from alkene. In the present specification, the alkenediyl group may be either linear or branched. In the present specification, the number of olefinic carbon-carbon double bonds in the alkene or alkenediyl group may be either one or two or more. Examples of the alkenediyl group include ethenediyl group, propenediyl group, butenediyl group, pentenediyl group, hexenediyl group, heptenediyl group, octenediyl group, nonenediyl group, and decenediyl group. In the present specification, the "compound name + diyl group (e.g., ethenediyl group)" refers to a divalent group having a structure obtained by removing two hydrogen atoms from the aforementioned compound.

In the present specification, the "alkynediyl group" refers to a divalent group having a structure obtained by removing two hydrogen atoms from alkyne. In the present specification, the alkynediyl group may be either linear or branched. In the present specification, the number of carbon-carbon triple bonds in the alkynediyl group may be only one, or two or more. Examples of the alkynediyl group include ethynediyl group, propynediyl group, butynediyl group, pentynediyl group, hexynediyl group, heptynediyl group, octynediyl group, nonynediyl group, and decynediyl group.

In the formula (5), R¹¹ is preferably an alkylene group having 2 to 9 carbon atoms, an alkenediyl group having 2 to 9 carbon atoms, or an alkynediyl group having 2 to 9 carbon atoms, more preferably an alkylene group having 2 to 9 carbon atoms or an alkenediyl group having 2 to 9 carbon atoms, further preferably an alkylene group having 2 to 9 carbon atoms, particularly preferably an alkylene group having 2 or 3 carbon atoms, most preferably a trimethylene group.

In the formula (5), R¹² and R¹³ are each independently an alkyl group having 1 to 17 carbon atoms, an alkenyl group having 2 to 17 carbon atoms, or an alkynyl group having 2 to 17 carbon atoms; at least one ethylene group or at least one trimethylene group in R¹² may be replaced with at least one bond selected from the group consisting of an ester bond, an amide bond, a carbamate bond, and a carbonate bond; at least one ethylene group or at least one trimethylene group in R¹³ may be replaced with at least one bond selected from the group consisting of an ester bond, an amide bond, a carbamate bond, and a carbonate bond; and R¹² and R¹³ are each independently optionally substituted by a substituent selected from the group consisting of a halogen atom, a hydroxyl group, and a hydrocarbon ring group having 3 to 12 carbon atoms.

R¹² and R¹³ are preferably each independently an alkyl group having 1 to 17 carbon atoms, an alkenyl group having 2 to 17 carbon atoms, or an alkynyl group having 2 to 17 carbon atoms, more preferably each independently an alkyl group having 1 to 17 carbon atoms, further preferably an alkyl group having 1 to 10 carbon atoms, particularly preferably a hexyl group.

In the formula (5), X⁴ is an oxygen atom, NH, or a sulfur atom. X⁴ is preferably an oxygen atom or NH, more preferably an oxygen atom.

In the formula (5), it is most preferred that R¹¹ is a trimethylene group, R¹² and R¹³ are both hexyl groups, and X⁴ is an oxygen atom.

R^{3a} may be the same as or different from R^{3b}, and R^{3a} is preferably the same group as R^{3b}.

In one embodiment of the present invention, R^{1a} is the same as R^{1b}, X² is the same as X^{b}, R^{2a} is the same as R^{2b}, Y^{a} is the same as Y^{b}, Z^{a} is the same as Z^{b}, and R^{3a} is the same as R^{3b}.

Preferred examples of the ionic lipid represented by the formula (1) (sometimes to be abbreviated as "ionic lipid (1)" in the present specification) include the following ionic lipids.

### [Ionic lipid (1-1a)]

Ionic lipid (1) wherein
R^{1a} and R^{1b} are each independently an alkylene group having 1 to 6 carbon atoms (e.g., methylene group, ethylene group);
X^{a} and X^{b} are each independently an acyclic alkyl tertiary amino group having 1 to 6 carbon atoms and one tertiary amino group (e.g., -N(CH₃)-), or a cyclic alkylene tertiary amino group having 2 to 5 carbon atoms and one or two tertiary amino groups (e.g., piperidylene group);
R^{2a} and R^{2b} are each independently an alkylene group having 8 or less carbon atoms (e.g., methylene group, ethylene group, trimethylene group);
Y^{a} and Y^{b} are each independently an ester bond or amide bond;
Z^{a} and Z^{b} are each independently a divalent group derived from an aromatic compound having 3 to 16 carbon atoms, at least one aromatic ring, and optionally having a heteroatom (e.g., - C₆H₄-CH₂-, -CH₂-C₆H₄-CH₂-) ;
n^{a} and n^{b} are each independently 0 or 1;
R^{3a} and R^{3b} are each independently a residue derived from a reaction product of a liposoluble vitamin having a hydroxyl group (e.g., tocopherol) and succinic anhydride or glutar acid anhydride, an aliphatic hydrocarbon group having 12 to 22 carbon atoms (e.g., heptadecenyl group, heptadecadienyl group, 1-hexylnonyl group), a group having 50 or less carbon atoms and represented by the aforementioned formula (4) (in the formula (4), R¹⁰ is an alkyl group having 1 to 20 carbon atoms), or a group having 50 or less carbon atoms and represented by the aforementioned formula (5) (in the formula (5), R¹¹ is an alkylene group having 2 to 9 carbon atoms, R¹² and R¹³ are each independently an alkyl group having 1 to 17 carbon atoms, and X⁴ is an oxygen atom or NH).

### [Ionic lipid (1-1b)]

Ionic lipid (1) wherein
R^{1a} and R^{1b} are each independently an alkylene group having 1 to 6 carbon atoms (e.g., methylene group, ethylene group);
X^{a} and X^{b} are each independently an acyclic alkyl tertiary amino group having 1 to 6 carbon atoms and one tertiary amino group (e.g., -N(CH₃)-), or a cyclic alkylene tertiary amino group having 2 to 5 carbon atoms and one or two tertiary amino groups (e.g., piperidylene group);
R^{2a} and R^{2b} are each independently an alkylene group having 8 or less carbon atoms (e.g., methylene group, ethylene group, trimethylene group);
Y^{a} and Y^{b} are each independently an ester bond or amide bond;
Z^{a} and Z^{b} are each independently a divalent group derived from an aromatic compound having 3 to 16 carbon atoms, at least one aromatic ring, and optionally having a heteroatom (e.g., - C₆H₄-CH₂-, -CH₂-C₆H₄-CH₂-) ;
n^{a} and n^{b} are each independently 0 or 1; and
R^{3a} and R^{3b} are each independently a residue derived from a reaction product of a liposoluble vitamin having a hydroxyl group (e.g., tocopherol) and succinic anhydride or glutar acid anhydride, or an aliphatic hydrocarbon group having 12 to 22 carbon atoms (e.g., heptadecenyl group, heptadecadienyl group, 1-hexylnonyl group).

### [ionic lipid (1-2a)]

Ionic lipid (1) wherein
R^{1a} and R^{1b} are each independently an alkylene group having 1 to 4 carbon atoms (e.g., methylene group, ethylene group);
X^{a} and X^{b} are each independently an acyclic alkyl tertiary amino group having 1 to 3 carbon atoms and one tertiary amino group (e.g., -N(CH₃)-), or a cyclic alkylene tertiary amino group having 2 to 5 carbon atoms and one or two tertiary amino groups (e.g., piperidylene group);
R^{2a} and R^{2b} are each independently an alkylene group having 6 or less carbon atoms (e.g., methylene group, ethylene group, trimethylene group);
Y^{a} and Y^{b} are each independently an ester bond or amide bond;
Z^{a} and Z^{b} are each independently a divalent group derived from an aromatic compound having 6 to 12 carbon atoms, one aromatic ring, and optionally having a heteroatom (e.g., -C₆H₄-CH₂-, -CH₂-C₆H₄-CH₂-) ;
n^{a} and n^{b} are each independently 0 or 1;
R^{3a} and R^{3b} are each independently a residue derived from a reaction product of a liposoluble vitamin having a hydroxyl group (e.g., tocopherol) and succinic anhydride, an aliphatic hydrocarbon group having 13 to 19 carbon atoms (e.g., heptadecenyl group, heptadecadienyl group, 1-hexylnonyl group), a group having 50 or less carbon atoms and represented by the aforementioned formula (4) (in the formula (4), R¹⁰ is an alkyl group having 1 to 10 carbon atoms), or a group having 50 or less carbon atoms and represented by the aforementioned formula (5) (in the formula (5), R¹¹ is an alkylene group having 2 or 3 carbon atoms, R¹² and R¹³ are each independently an alkyl group having 1 to 10 carbon atoms, and X⁴ is an oxygen atom).

### [Ionic lipid (1-2b)]

Ionic lipid (1) wherein
R^{1a} and R^{1b} are each independently an alkylene group having 1 to 4 carbon atoms (e.g., methylene group, ethylene group);
X^{a} and X^{b} are each independently an acyclic alkyl tertiary amino group having 1 to 3 carbon atoms and one tertiary amino group (e.g., -N(CH₃)-), or a cyclic alkylene tertiary amino group having 2 to 5 carbon atoms and one tertiary amino group (e.g., piperidylene group);
R^{2a} and R^{2b} are each independently an alkylene group having 6 or less carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 6 to 12 carbon atoms and one aromatic ring, and optionally having a hetero atom (e.g., - C₆H₄-CH₂-, -CH₂-C₆H₄-CH₂-) ;
n^{a} and n^{b} are each independently 0 or 1; and
R^{3a} and R^{3b} are each independently a residue derived from a reaction product of liposoluble vitamin having a hydroxyl group (e.g., tocopherol) and succinic anhydride or an aliphatic hydrocarbon group having 13 to 19 carbon atoms (e.g., heptadecenyl group, heptadecadienyl group, 1-hexylnonyl group).

### [Ionic lipid (1-3a)]

Ionic lipid (1) wherein
R^{1a} and R^{1b} are each independently an alkylene group having 1 to 2 carbon atoms (i.e., methylene group or ethylene group);
X^{a} and X^{b} are each independently X¹: 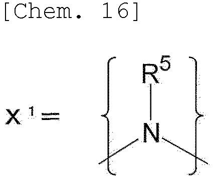 wherein R⁵ is an alkyl group having 1 to 3 carbon atoms (e.g., methyl group), or X²: 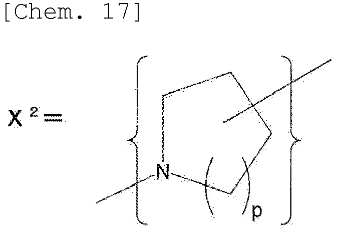 wherein p is 1 or 2;
R^{2a} and R^{2b} are each independently alkylene group having 4 or less carbon atoms (e.g., methylene group, ethylene group, trimethylene group);
Y^{a} and Y^{b} are each independently an ester bond or an amide bond;
Z^{a} and Z^{b} are each independently Z¹: 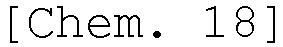 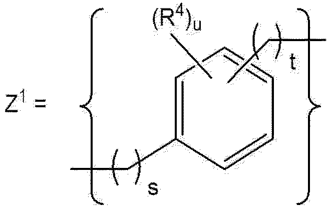 wherein s is an integer of 0 to 1, t is an integer of 0 to 2, u is an integer of 0 to 2 (preferably 0), and R⁴ in the number of u are each independently a substituent;
n^{a} and n^{b} are each independently 0 or 1; and
R^{3a} and R^{3b} are each independently a residue derived from a reaction product of a liposoluble vitamin having a hydroxyl group (e.g., tocopherol) and succinic anhydride, an aliphatic hydrocarbon group having 13 to 17 carbon atoms (e.g., heptadecenyl group, heptadecadienyl group, 1-hexylnonyl group), a group having 50 or less carbon atoms and represented by the aforementioned formula (4) (in the formula (4), R¹⁰ is an alkyl group having 1 to 10 carbon atoms), or a group having 50 or less carbon atoms and represented by the aforementioned formula (5) (in the formula (5), R¹¹ is an alkylene group having 2 or 3 carbon atoms, R¹² and R¹³ are each independently an alkyl group having 1 to 10 carbon atoms, and X⁴ is an oxygen atom).

### [Ionic lipid (1-3b)]

Ionic lipid (1) wherein
R^{1a} and R^{1b} are each independently an alkylene group having 1 to 2 carbon atoms (i.e., methylene group or ethylene group);
X^{a} and X^{b} are each independently X¹: 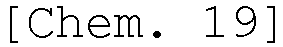 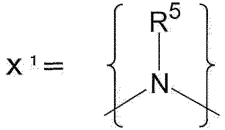 wherein R⁵ is an alkyl group having 1 to 3 carbon atoms (e.g., methyl group), or X²: 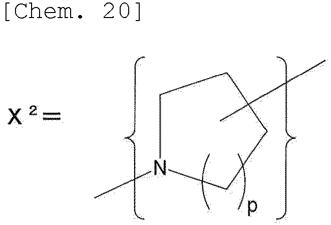 wherein p is 1 or 2;
R^{2a} and R^{2b} are each independently alkylene group having 4 or less carbon atoms (e.g., methylene group, ethylene group, trimethylene group);
Y^{a} and Y^{b} are each independently an ester bond or an amide bond;
Z^{a} and Z^{b} are each independently Z¹: 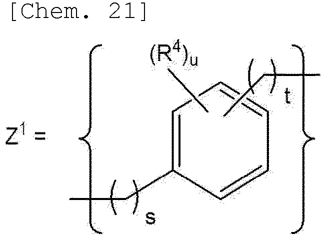 wherein s is an integer of 0 to 1, t is an integer of 0 to 2, u is an integer of 0 to 2 (preferably 0), and R⁴ in the number of u are each independently a substituent;
n^{a} and n^{b} are each independently 0 or 1; and
R^{3a} and R^{3b} are each independently a residue derived from a reaction product of a liposoluble vitamin (e.g., tocopherol) having a hydroxyl group and succinic anhydride or an aliphatic hydrocarbon group having 13 to 17 carbon atoms (e.g., heptadecenyl group, heptadecadienyl group, 1-hexylnonyl group).

Specific examples of ionic lipid (1) include the following O-Ph-P3C1, O-Ph-P4C1, O-Ph-P4C2, O-Bn-P4C2, E-Ph-P4C2, L-Ph-P4C2, HD-Ph-P4C2, O-Ph-amide-P4C2, O-Ph-C3M, B-2, B-2-5, TS-P4C2, L-P4C2, O-P4C2, PiP9, and PiP15.

**[Table 1-1]**

| name of ionic lipid | structure |
|---|---|
| O-Ph-P3C1 | 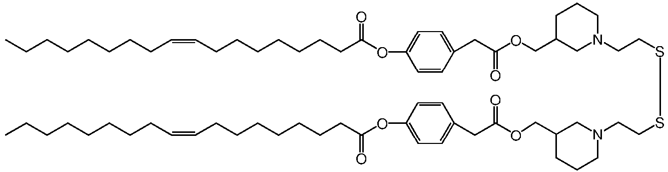 |
| O-Ph-P4C1 | 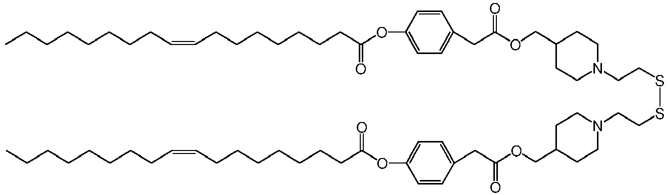 |
| O-Ph-P4C2 (or SS-OP) | 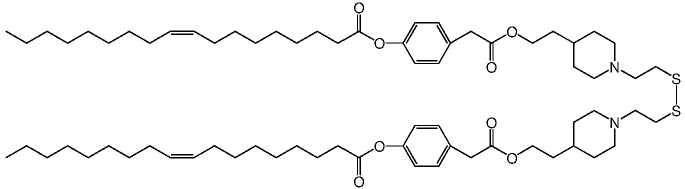 |
| O-Bn-P4C2 | 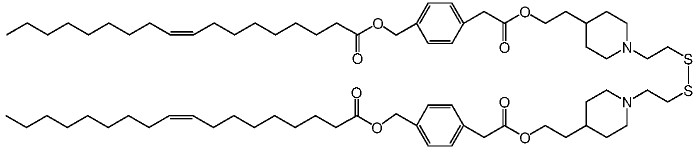 |
| E-Ph-P4C2 (or SS-EP) | 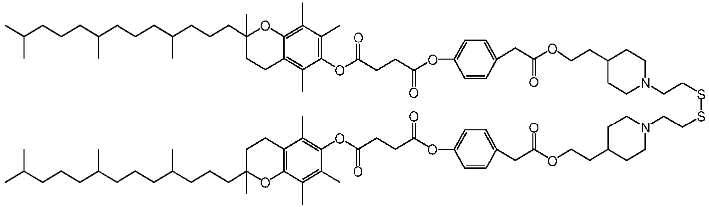 |

**[Table 1-2]**

| name of ionic lipid | structure |
|---|---|
| L-Ph-P4C2 | 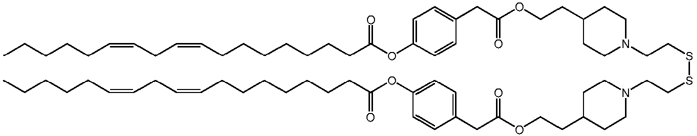 |
| HD-Ph-P4C2 | 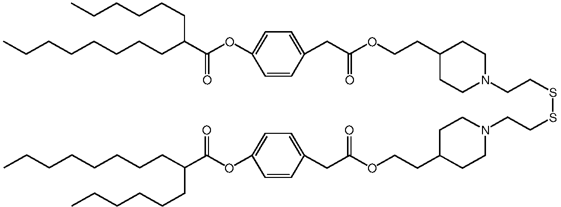 |
| O-Ph-amide-P4C2 | 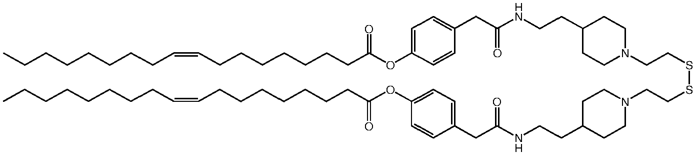 |
| O-Ph-C3M | 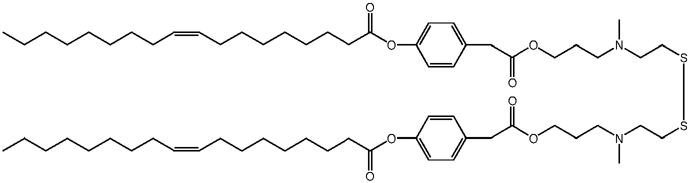 |

**[Table 1-3]**

| name of ionic lipid | structure |
|---|---|
| B-2 | 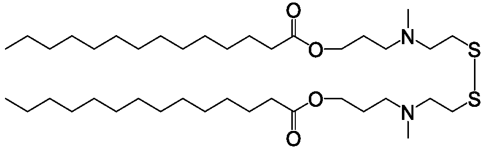 |
| B-2-5 | 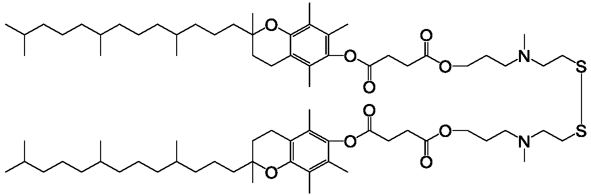 |
| TS-P4C2 (or SS-EC) | 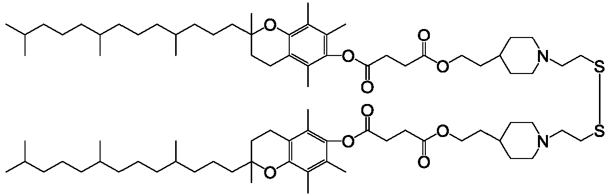 |
| L-P4C2 | 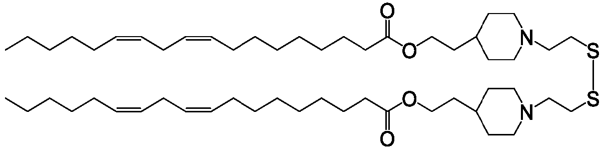 |
| O-P4C2 (or SS-OC) | 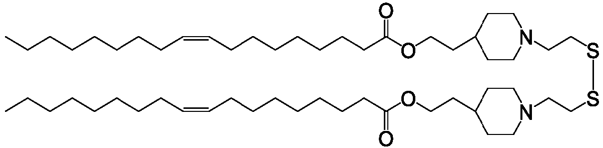 |

**[Table 1-4]**

| name of ionic lipid | structure |
|---|---|
| PiP9 | 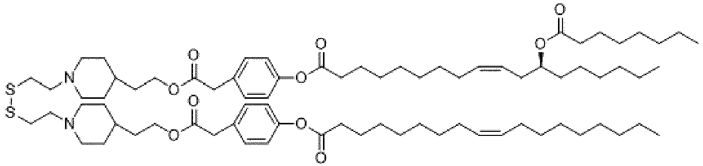 |
| PiP15 | 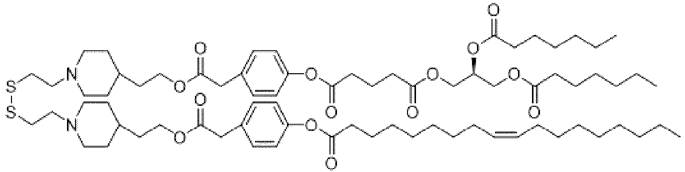 |

Ionic lipid (1) is preferably at least one selected from the group consisting of the compounds listed in the aforementioned Table 1-1 to Table 1-4, and more preferably at least one selected from the group consisting of SS-OP, SS-EC, PiP9, and PiP15.

Ionic lipid (1) is further preferably (i) SS-OP, (ii) SS-OP and SS-EC, (iii) PiP9, or (iv) PiP15.

Ionic lipid (1) is particularly preferably (i) SS-OP or (ii) SS-OP and SS-EC. Ionic lipid (1) is most preferably SS-OP.

The ionic lipid (1) can be produced by a known method (e.g., the method described in WO 2019/188867 A1 (US2021/0023008A1), US 9708628 B2, WO 2021/195529 A2). The PiP9 and PiP15 used in the Examples were produced by the following synthesis method. An ionic lipid (1) other than PiP9 or PiP15 can also be produced in the same manner as in the synthesis method for PiP9 or PiP15.

### (I) Synthesis method of PiP9

### <Synthesis of intermediate 1>

Intermediate 1 was synthesized by the following synthesis route.

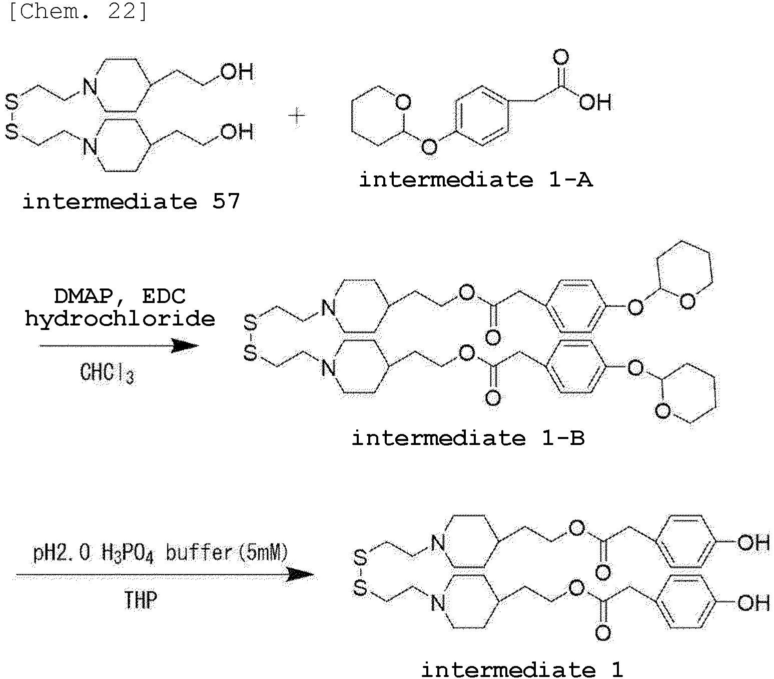

### <Synthesis of intermediate 1-A>

4-Hydroxyphenylacetic acid (30.0 g, 197 mmol) and pyridinium p-toluenesulfonate (5.00 g, 19.9 mmol) were dissolved in dichloromethane (120 mL) at room temperature. To the obtained mixture was added dropwise a mixed solution of 3,4-dihydro-2H-pyran (83.0 g, 987 mmol) and dichloromethane (31.1 mL) at 20°C or less and the mixture was reacted at room temperature for 2 hr. Thereafter, the reaction mixture was neutralized by adding DMAP (12.0 g, 98.2 mmol). To the obtained mixture were added 2-propanol (301 mL) and 100 g/L NaOH aqueous solution (160 g), and the mixture was reacted at room temperature for 1 hr. The mixed solution was concentrated with an evaporator, and the concentrate was washed with chloroform and neutralized by adding 6 M hydrochloric acid. The obtained mixture was extracted with chloroform, and sodium sulfate was added to the organic layer for dehydration. Sodium sulfate was removed by filtration, and the filtrate was concentrated with an evaporator to obtain 47.0 g of intermediate 1-A.

### <Synthesis of intermediate 1-B>

Intermediate 57 (36.0 g, 95.6 mmol) synthesized according to the method described in WO 2016/121942, intermediate 1-A (49.7 g, 210 mmol), and DMAP (4.68 mg, 38.3 mmol) were dissolved in 240 mL of chloroform at room temperature. To the obtained mixture was added EDC hydrochloride (55.1 g, 287 mmol) and the mixture was reacted at room temperature for 2 hr. Thereafter, the reaction solution was washed with 5 wt% sodium dihydrogen phosphate aqueous solution, 9 wt% sodium hydrogen carbonate solution, and 20 wt% brine, and then sodium sulfate was added for dehydration. Sodium sulfate was removed by filtration, and the filtrate was concentrated with an evaporator to obtain 77.9 g of intermediate 1-B.

### <Synthesis of intermediate 1>

Intermediate 1-B (77.7 g, 95.6 mmol) was dissolved in 325 mL of THF at room temperature. To the obtained mixture were added 2-propanol (319 mL) and p-toluenesulfonic acid monohydrate (38.2 g, 201 mmol) and the mixture was reacted at 25°C or less for 1 hr. Thereafter, the reaction mixture was neutralized by adding DMAP (25.0 g, 205 mmol). DMAP was removed by filtration, and the filtrate was concentrated with an evaporator. The obtained residue was dissolved in 627 mL of chloroform, washed with 0.5 M phosphate buffer (pH=6.5) and 0.5 M glycine buffer (pH=9.5), and then dehydrated by adding sodium sulfate. Sodium sulfate was removed by filtration and 1.56 L of toluene was added for crystallization. The obtained crude product was washed with hexane and then vacuum dried to obtain 40.3 g of intermediate 1.

### <Synthesis of intermediate 1-1>

Oleic acid (5.11 g, 18.1 mmol), intermediate 1 (16.6 g, 25.8 mmol), and DMAP (630 mg, 5.16 mmol) were dissolved in chloroform (166 mL) at room temperature. To the obtained mixture was added EDC hydrochloride (5.94 g, 31.0 mmol) and the mixture was reacted at room temperature for 2 hr. Thereafter, the reaction solution was washed with 5 wt% sodium hydrogenphosphate aqueous solution, 0.5 M phosphate buffer (pH=2.0), and 20 wt% brine, and sodium sulfate was added for dehydration. Sodium sulfate was removed by filtration, and the filtrate was concentrated with an evaporator. The obtained residue was purified by silica gel column chromatography (chloroform/ethanol=88/12) to obtain 8.21 g of intermediate 1-1.

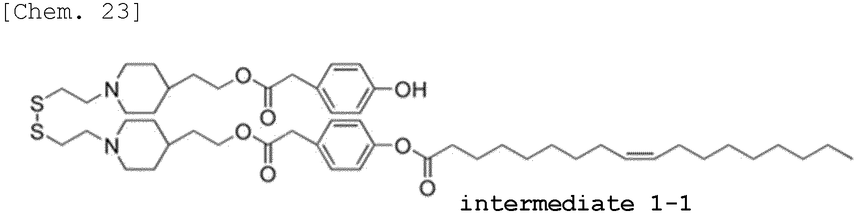

### <Synthesis of intermediate 5-1>

Methyl ricinoleate (5.00 g, 16.0 mmol), octanoic acid (2.54 g, 17.6 mmol), and DMAP (391 mg, 3.20 mmol) were dissolved in chloroform (50.0 g) at room temperature. To the obtained mixture was added EDC hydrochloride (4.60 g, 24.0 mmol)and the mixture was reacted at room temperature for 1 hr. Thereafter, the reaction solution was washed with 0.5 M phosphate buffer (pH 4.0), 7 wt% sodium bicarbonate water, and 20 wt% brine, and sodium sulfate was added for dehydration. Sodium sulfate was removed by filtration, and the filtrate was concentrated with an evaporator to obtain 6.78 g of intermediate 5-1.

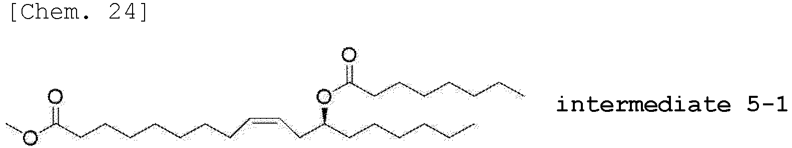

### <Synthesis of intermediate 5>

Intermediate 5-1 (6.40 g, 14.6 mmol) was dissolved in tert-butanol at room temperature to a concentration of 0.3 M. To the obtained mixture was added 2 M NaOH aqueous solution (8.17 g) and the mixture was reacted at room temperature for 8 hr. Thereafter, 1 M hydrochloric acid was added, and the obtained mixture was extracted with hexane. The organic layer was washed with 20 wt% brine, and sodium sulfate was added for dehydration. Sodium sulfate was removed by filtration, and the filtrate was concentrated with an evaporator. The obtained residue was purified by silica gel column chromatography (chloroform/ethanol=94/6 (volume ratio)) to obtain 5.93 g of intermediate 5.

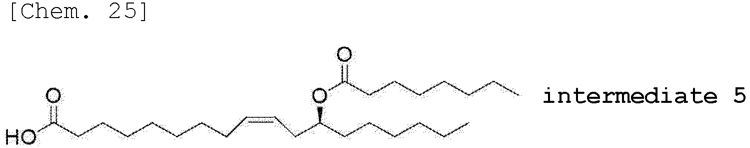

### <Synthesis of PiP9>

Intermediate 1-1 (492 mg, 0.541 mmol) was dissolved by adding chloroform (2.91 g), intermediate 5 (230 mg, 0.541 mmol), DMAP (13.2 mg, 0.108 mmol), and EDC hydrochloride (207 mg, 1.08 mmol) were added, and the mixture was reacted at room temperature for 2 hr. The solution after the reaction was washed with 5 wt% brine and concentrated with an evaporator. After concentration, silica gel column purification was performed using ethanol/chloroform to obtain 532 mg of PiP9.

### (II) Synthesis method of PiP15

### <Synthesis of intermediate 11-1>

(R)-(-)-2,2-dimethyl-1,3-dioxolane-4-methanol (25.0 g, 189 mmol) and imidazole (19.3 g, 284 mmol) were dissolved in dimethylformamide (251 g) at room temperature. To the obtained mixture was added TBDPS-Cl (57.3 g, 208 mmol) and the mixture was reacted at room temperature for 1 hr. Thereafter, chloroform was added to the reaction solution, and the mixture was washed with 5 wt% sodium hydrogenphosphate aqueous solution and ion exchange water, and sodium sulfate was added for dehydration. Sodium sulfate was removed by filtration, and the filtrate was concentrated with an evaporator to obtain 56.0 g of intermediate 11-1.

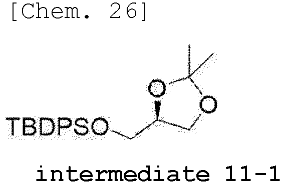

### <Synthesis of intermediate 11-2>

Intermediate 11-1 (55.9 g, 151 mmol) and 0.5 M phosphate buffer (pH1.0) (503 g) were dissolved in 530 g of THF at room temperature and reacted at 50°C for 12 hr. 1.0 M NaOH aqueous solution was added to pH 7.0. To the obtained mixture was added chloroform, and the mixture was washed with 20 wt% brine, and sodium sulfate was added for dehydration. Sodium sulfate was removed by filtration, and the filtrate was concentrated with an evaporator to obtain 44.9 g of intermediate 11-2.

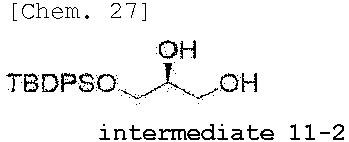

### <Synthesis of intermediate 14-1>

Intermediate 11-2 (6.90 g, 20.9 mmol), heptanoic acid (6.84 g, 46.0 mmol), and DMAP (0.511 g, 4.18 mmol) were dissolved in chloroform (69.5 g) at room temperature. To the obtained mixture was added EDC hydrochloride (12.0 g, 62.7 mmol) and the mixture was reacted at room temperature for 1 hr. Thereafter, the reaction solution was washed with 0.5 M phosphate buffer (pH 4.0), 7 wt% sodium bicarbonate water, and 20 wt% brine, and sodium sulfate was added for dehydration. Sodium sulfate was removed by filtration, and the filtrate was concentrated with an evaporator to obtain 9.03 g of intermediate 14-1.

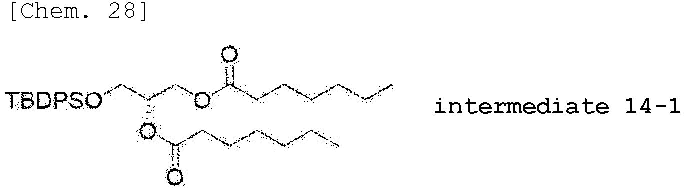

### <Synthesis of intermediate 14-2>

Intermediate 14-1 (9.41 g, 17.9 mmol) and acetic acid (3.92 g, 65.3 mmol) were dissolved in THF (27.2 g) at room temperature. To the obtained mixture was added 1 M TBAF THF solution (59.9 g, 65.2 mmol) and the mixture was reacted at room temperature for 18 hr. Thereafter, ethyl acetate (94.3 g) was added to the reaction solution. Thereafter, the reaction solution was washed with 0.5 M phosphate buffer (pH 4.0) and 20 wt% brine, and sodium sulfate was added for dehydration. Sodium sulfate was removed by filtration and the filtrate was concentrated with an evaporator to obtain 4.90 g of intermediate 14-2.

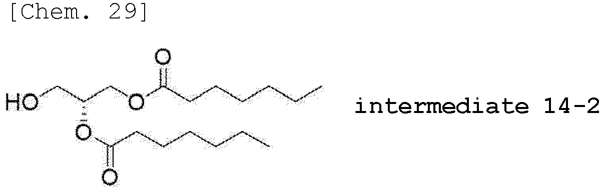

### <Intermediate 14>

Intermediate 14-2 (4.90 g, 15.4 mmol), glutar acid anhydride (3.73 g, 32.7 mmol), triethylamine (4.95 g, 48.9 mmol), and DMAP (0.398 g, 3.26 mmol) were dissolved in chloroform (51.6 g) at room temperature, and the mixture was reacted at room temperature for 1 hr. Thereafter, the reaction solution was washed with 0.5 M phosphate buffer (pH 4.0), 7 wt% sodium bicarbonate water, and 20 wt% brine, and sodium sulfate was added for dehydration. Sodium sulfate was removed by filtration, and the filtrate was concentrated with an evaporator to obtain 3.16 g of intermediate 14.

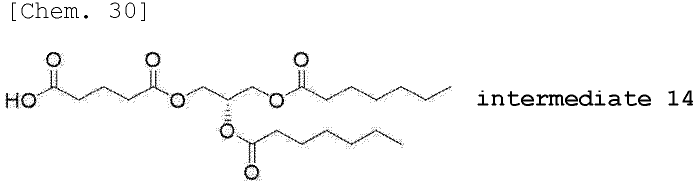

### <Synthesis of PiP15>

Using intermediate 14 and intermediate 1-1 and in the same manner as in the aforementioned synthesis of PiP9, 0.215 g of PiP15 was synthesized.

The content of ionic lipid (1) in the lyophilized composition is preferably 20 to 75 mol%, more preferably 30 to 70 mol%, further preferably 35 to 60 mol%, based on the total lipids, from the aspects of nucleic acid encapsulation efficiency, intracellular release efficiency of nucleic acid, and stability of lipid nanoparticles.

### Nucleic acid

Examples of nucleic acids to be encapsulated in lipid nanoparticles include those described below. The nucleic acid is preferably mRNA.

The molar ratio of the total amino groups of ionic lipid (1) to the phosphate groups of nucleic acid (total amino groups of ionic lipid/phosphate groups of nucleic acid) (sometimes referred to as "N/P ratio" in the present specification) is preferably 5 to 280, more preferably 10 to 140, further preferably 15 to 70, from the aspects of nucleic acid encapsulation efficiency and toxicity.

### Phospholipid

Phospholipids can be used as a lipid membrane constituting component of lipid nanoparticles.

Examples of the phospholipid include 1,2-diacyl-sn-glycero-3-phosphocholine (PC), 1,2-diacyl-sn-glycero-3-phosphoethanolamine (PE), 1,2-diacyl-sn-glycero-3-phosphoserine (PS), 1,2-diacyl-sn-glycero-3-phosphoglycerol (PG), 1,2-diacyl-sn-glycero-3-phosphatidic acid (PA), and lyso forms of these.

Specific examples of 1,2-diacyl-sn-glycero-3-phosphocholine (PC) include 1,2-didecanoyl-sn-glycero-3-phosphocholine (DDPC), 1,2-dilauroyl-sn-glycero-3-phosphocholine (DLPC), 1,2-dimyristoyl-sn-glycero-3-phosphocholine (DMPC), 1,2-dipalmitoyl-sn-glycero-3-phosphocholine (DPPC), 1,2-distearoyl-sn-glycero-3-phosphocholine (DSPC), 1,2-dioleoyl-sn-glycero-3-phosphocholine (DOPC), 1,2-dilinoleoyl-sn-glycero-3-phosphocholine (DLoPC), 1,2-dierucoyl-sn-glycero-3-phosphocholine (DEPC), 1-myristoyl-2-palmitoyl-sn-glycero-3-phosphocholine (MPPC), 1-myristoyl-2-stearoyl-sn-glycero-3-phosphocholine (MSPC), 1-palmitoyl-2-myristoyl-sn-glycero-3-phosphocholine (PMPC), 1-palmitoyl-2-stearoyl-sn-glycero-3-phosphocholine (PSPC), 1-palmitoyl-2-oleoyl-sn-glycero-3-phosphocholine (POPC), 1-stearoyl-2-oleoyl-sn-glycero-3-phosphocholine (SOPC).

It is also possible to use 1,2-diacyl-sn-glycero-3-phosphoethanolamine (PE), 1,2-diacyl-sn-glycero-3-phosphoserine (PS), 1,2-diacyl-sn-glycero-3-phosphoglycerol (PG), and 1,2-diacyl-sn-glycero-3-phosphatidic acid (PA), in which the phosphocholine moiety of the above-mentioned specific examples of 1,2-diacyl-sn-glycero-3-phosphocholine (PC) is substituted by phosphoethanolamine, phosphoserine, phosphoglycerol, and phosphatidic acid, respectively.

Phospholipid to be used in the present invention is preferably PC or PE, further preferably DOPC, DSPC, DEPC, POPC, DOPE (1,2-dioleoyl-sn-glycero-3-phosphoethanolamine), or POPE (1-palmitoyl-2-oleoyl-sn-glycero-3-phosphoethanolamine), particularly preferably DOPC, DSPC, or DEPC.

The content of phospholipid in the lyophilized composition is preferably 0 to 35 mol%, more preferably 2.5 to 30 mol%, further preferably 5 to 30 mol%, with respect to the total lipids, from the aspects of efficiency of nucleic acid encapsulation, efficiency of intracellular release of nucleic acid, and stability of the lipid nanoparticles.

### Sterol

Sterol can be used as a component that regulates fluidity of the lipid membrane of lipid nanoparticles. Examples of the sterol include cholesterol, lanosterol, phytosterol, zymosterol, zymostenol, desmosterol, stigmastanol, dihydrolanosterol, and 7-dehydrocholesterol. Sterol is preferably cholesterol, lanosterol, or phytosterol, further preferably cholesterol.

When sterol is used, the content thereof in the lyophilized composition is preferably 5 to 60 mol%, more preferably 7 to 55 mol%, further preferably 10 to 50 mol%, with respect to the total lipids, from the aspects of efficiency of nucleic acid encapsulation, efficiency of intracellular release of nucleic acid, and stability of the lipid nanoparticles.

### PEG lipid

PEG lipids can be used as stabilizers that coat the surface of lipid nanoparticles with hydrophilic polyethylene glycol (PEG) and suppress the aggregation of particles, or used to suppress the interaction between biological components and particles when administered to a living body.

The PEG region can have any molecular weight. In some embodiments, the PEG region has a number average molecular weight of 200 to 10,000 and may be linear or branched.

Examples of the PEG lipid include PEG-phospholipid, PEG-ceramide, PEG-diacylglycerol, and PEG-cholesterol. It is preferably a diacylglycerol PEG having a PEG number molecular weight of 1,000 to 10,000, further preferably a dimyristoylglycerol PEG or a distearoylglycerol PEG each having a PEG number molecular weight of 1,000 to 10,000.

When PEG lipid is used, the content thereof in the lyophilized composition is preferably 0.1 to 6.0 mol %, more preferably 0.5 to 5.0 mol %, further preferably 0.7 to 3.0 mol %, with respect to the total of other lipids, from the aspects of efficiency of nucleic acid encapsulation, efficiency of intracellular release of nucleic acid, and stability of the lipid nanoparticles. As used herein, "other lipid" means lipids other than PEG lipids. For example, when ionic lipid (1) and PEG lipid are used as the lipids, "total of other lipids" means ionic lipid (1). When ionic lipid (1), phospholipid, sterol, and PEG lipid are used as the lipids, "total of other lipids" means the total of ionic lipid (1), phospholipid, and sterol. In the present specification, the "content of B (mol%) with respect to A" means the "100 × amount of B (mol)/amount of A (mol)".

### Other component

The lyophilized composition of nucleic acid-encapsulating lipid nanoparticles of the present invention may contain other components other than the nucleic acid and lipids as long as the effects of the present invention are not impaired. Only one kind of other component may be used, or two or more kinds thereof may be used in combination.

### Cryoprotectant

As other component, one or more kinds of cryoprotectants may be used. Examples of the cryoprotectant include saccharides such as monosaccharide, sugar alcohol, disaccharide, oligosaccharide, polysaccharide, and the like. The saccharide is preferably disaccharide, further preferably sucrose.

When a cryoprotectant is used, the content thereof in the lyophilized composition is preferably 90.0 to 99.9 wt%, more preferably 95.0 to 99.9 wt%, further preferably 97.0 to 99.9 mol%, with respect to the total lyophilized composition, from the aspects of stability of the lipid nanoparticles, freeze protection ability, and particle formation.

### Production method of lyophilized composition

The present invention provides a method for producing a lyophilized composition of nucleic acid-encapsulating lipid nanoparticles, including the following steps:
a) mixing an alcohol solution comprising an ionic lipid represented by the formula (1), a sterol, a phospholipid, and a PEG lipid with a nucleic acid solution in an acidic buffer having a buffering action at pH 1.0 to 6.5 to prepare a suspension of nucleic acid-encapsulating lipid nanoparticles;
b) exchanging an external aqueous phase of the nucleic acid-encapsulating lipid nanoparticles with another buffer having a buffering action at pH 4.5 to 6.9 to obtain a mixture comprising the nucleic acid-encapsulating lipid nanoparticles at pH 4.5 to 6.9;
c) mixing the obtained mixture with a cryoprotectant to obtain a mixture comprising 80 to 800 mg/mL of the cryoprotectant and nucleic acid-encapsulating lipid nanoparticles at pH 4.5 to 6.9;
d) lyophilizing the mixture obtained in step c) to obtain a lyophilized composition.

### a) step of preparing suspension of nucleic acid-encapsulating lipid nanoparticles

In the method of the present invention, a suspension of nucleic acid-encapsulating lipid nanoparticles is prepared by mixing an alcohol solution containing ionic lipid (1), phospholipid, sterol, and PEG lipid (hereinafter sometimes to be abbreviated as "alcohol solution") with a nucleic acid solution in an acidic buffer having a buffering action at pH 1.0 to 6.5 (hereinafter sometimes to be abbreviated as "acidic buffer") (hereinafter sometimes to be abbreviated as "nucleic acid solution"). In order to prepare lipid nanoparticles, any operation that induces organization can be used. Examples of alcohol include ethanol, tert-butanol, and the like.

The concentration of total lipid (i.e., the total of ionic lipid (1), phospholipid, sterol, and PEG lipid) in the alcohol solution used in step a) is preferably 0.1 to 30 mM, more preferably 0.5 to 25 mM, further preferably 1 to 20 mM, from the aspects of lipid solubility and nucleic acid encapsulation.

The concentration of ionic lipid (1) in the alcohol solution used in step a) is preferably 0.005 to 30 mM, more preferably 0.05 to 25 mM, further preferably 0.1 to 20 mM, from the aspect of lipid solubility.

The concentration of sterol in the alcohol solution used in step a) is preferably 0.001 to 25 mM, more preferably 0.005 to 20 mM, further preferably 0.01 to 15 mM, from the aspect of lipid solubility.

The concentration of phospholipid in the alcohol solution used in step a) is preferably 0.001 to 25 mM, more preferably 0.001 to 15 mM, further preferably 0.01 to 10 mM, from the aspect of lipid solubility.

The concentration of PEG lipid in the alcohol solution used in step a) is preferably 0.00001 to 10 mM, more preferably 0.0001 to 5.0 mM, further preferably 0.001 to 3.0 mM, from the aspect of lipid solubility.

In step a), an acidic buffer having a buffering action at pH 1.0 to 6.5 (preferably pH 3 to 6.5, more preferably pH 3 to 5.0) is used. In the present specification, the "acidic buffer having a buffering action at pH 1.0 to 6.5" means an acidic buffer having a pH in the range of 1.0 to 6.5 and having a buffering action that maintains the aforementioned pH. Expressions similar to the "acidic buffer having a buffering action at pH 1.0 to 6.5" also have meanings similar thereto.

Examples of acidic buffer include tartaric acid/NaOH buffer, phthalic acid HK/HCl buffer, glycine/HCl buffer, trans-aconitic acid/NaOH buffer, formic acid/Na formate buffer, 3,3-dimethylglutaric acid/NaOH buffer, 3,3-dimethylglutaric acid/NaOH/0.1M NaCl buffer, phenylacetic acid/Na phenylacetate buffer, acetic acid/Na acetate buffer, succinic acid/NaOH buffer, phthalic acid HK/NaOH buffer, Na cacodylate/HCl buffer, maleic acid HNa/NaOH buffer, maleic acid/Tris/NaOH buffer, phosphate buffer, KH₂PO₄/NaOH buffer, 2-morpholinoethanesulfonic acid (MES) buffer, malic acid buffer, phthalate buffer, maleate buffer, succinate buffer, tartrate buffer, citrate buffer, bis-Tris buffer, glycylglycine buffer, and the like.

The acidic buffer is preferably malic acid buffer, citrate buffer, or MES buffer (more preferably malic acid buffer or MES buffer) that has a buffering action at pH 1.0 to 6.5 (more preferably pH 3 to 6.5, further preferably pH 3 to 5.0).

The total concentration of the acid and a salt thereof in the acidic buffer used in step a) (hereinafter sometimes abbreviated as "buffer concentration") is preferably 10 to 100 mM, more preferably 10 to 80 mM, from the aspect of nucleic acid stability.

In the present specification, the "nucleic acid solution in an acidic buffer having a buffering action at pH 1.0 to 6.5" means a nucleic acid solution containing an acidic buffer having a buffering action at pH 1.0 to 6.5 as a solvent.

The concentration of nucleic acid in the nucleic acid solution used in step a) is preferably 0.1 to 720 µg/mL, more preferably 0.5 to 350 µg/mL, further preferably 1 to 250 µg/mL, from the aspect of nucleic acid solubility.

The volume ratio of the nucleic acid solution and the lipid alcohol solution when they are mixed (nucleic acid solution:lipid alcohol solution) is preferably 1:1 to 20:1, more preferably 1.5:1 to 15:1, further preferably 2:1 to 8:1, from the aspects of the particle size of the nucleic acid-encapsulating lipid nanoparticles and the nucleic acid encapsulation rate.

Examples of the "operation to induce organization" for preparing lipid nanoparticles include methods known per se such as alcohol dilution method using a microchannel or vortex, simple hydration method, sonication, heating, vortex, ether injecting method, French press method, cholic acid method, Ca²⁺ fusion method, freeze-thaw method, reversed-phase evaporation method, and the like. It is preferably an alcohol dilution method using a microchannel or vortex, further preferably an alcohol dilution method using a microchannel. Particles preparation in the alcohol dilution method using a microchannel can be performed using, for example, NanoAssemblr (Precision NanoSystems). The buffer in the external aqueous phase of the prepared lipid nanoparticles can be replaced by an operation such as ultrafiltration, dialysis, or dilution.

### b) step of obtaining mixture comprising nucleic acid-encapsulating lipid nanoparticles at pH 4.5 to 6.9

In step b), the external aqueous phase of the nucleic acid-encapsulating lipid nanoparticles is exchanged with another buffer (hereinafter sometimes to be abbreviated as "another buffer") having a buffering action at pH 4.5 to 6.9 (preferably pH 5.0 to 6.9, preferably pH 5.5 to 6.9) by an operation such as ultrafiltration, dialysis, or dilution. As used herein, the "external aqueous phase of the nucleic acid-encapsulating lipid nanoparticles" refers to the dispersion medium of the suspension of the nucleic acid-encapsulating lipid nanoparticles. The "another buffer having a buffering action at pH 4.5 to 6.9" means a buffer different from the acidic buffer used in step a), having a pH of 4.5 to 6.9, and having a buffering action that maintains the aforementioned pH. Expressions similar to the "another buffer having a buffering action at pH 4.5 to 6.9" also have meanings similar thereto.

The another buffer specifically includes tartaric acid/NaOH buffer, phthalic acid HK/HCl buffer, glycine/HCl buffer, trans-aconitic acid/NaOH buffer, formic acid/Na formate buffer, 3,3-dimethylglutaric acid/NaOH buffer, 3,3-dimethylglutaric acid/NaOH/0.1M NaCl buffer, phenylacetic acid/Na phenylacetate buffer, acetic acid/Na acetate buffer, succinic acid/NaOH buffer, phthalic acid HK/NaOH buffer, Na cacodylate/HCl buffer, maleic acid HNa/NaOH buffer, maleic acid/Tris/NaOH buffer, phosphate buffer, KH₂PO₄/NaOH buffer, MES buffer, malic acid buffer, phthalate buffer, maleate buffer, succinate buffer, tartrate buffer, citrate buffer, bis-Tris buffer, glycylglycine buffer, and the like.

The another buffer is preferably malic acid buffer, citrate buffer, MES buffer, or maleic acid/Tris/NaOH buffer (more preferably malic acid buffer or MES buffer) that has a buffering action at pH 4.5 to 6.9 (more preferably pH 5.0 to 6.9, further preferably pH 5.5 to 6.9).

The total concentration of the acid and a salt thereof in the another buffer (hereinafter sometimes abbreviated as "buffer concentration") is preferably 10 to 100 mM, more preferably 20 to 50 mM, from the aspects of nucleic acid encapsulation rate and lipid nanoparticle formation.

The concentration of nucleic acid in the mixture obtained in step b) is not particularly limited as long as it is a concentration at which lipid nanoparticles can be formed after exchange with another buffer. From the aspect of the particle size of the nucleic acid-encapsulating lipid nanoparticles, it is preferably 0.25 to 3300 µg/mL, more preferably 2.5 to 1500 µg/mL, further preferably 3 to 800 µg/mL. In step b), another buffer may be used in an amount that affords the above-mentioned nucleic acid concentration.

### c) step of mixing obtained mixture with cryoprotectant to obtain mixture comprising 80 to 320 mg/mL of cryoprotectant and nucleic acid-encapsulating lipid nanoparticles at pH 4.5 to 6.9

As a cryoprotectant, for example, saccharides such as monosaccharide, sugar alcohol, disaccharide, oligosaccharide, or polysaccharide can be added to a suspension of lipid nanoparticles. Saccharide is preferably disaccharide, further preferably sucrose. The concentration of a cryoprotectant (particularly saccharide) in the aforementioned mixture is 80 to 320 mg/mL, preferably 80 to 240 mg/mL, as a concentration before lyophilization.

### d) step of lyophilizing mixture obtained in step c) to obtain lyophilized composition

The lyophilization process can be performed in any suitable container known in the technical field of medicine, such as a glass container (or, for example, glass vial) or a two-chamber container.

The stabilized lipid nanoparticle composition of the present invention containing a cryoprotectant can be introduced into a glass container. The volume of the composition to be added to the container may be 0.1 to 20 mL, or 1 to 10 mL.

Any lyophilization process (including those known in the technical field of medicine) can be used. See, for example, Remington's Pharmaceutical Sciences, 18th Ed., Mack Publishing Co., Easton, Penn. (1990).

The lyophilization process may include freezing the lipid nanoparticle composition, stabilized by the cryoprotectant, at a temperature of about -55°C to about -30°C. The frozen composition may be a dried form of the lyophilized composition.

In some embodiments, the freezing step may include gradually lowering the temperature from room temperature to the final temperature over several minutes. The temperature gradient can be about 1°C/min.

In some embodiments, the drying step can be performed at a pressure of about 0 to 250 mTorr, or 50 to 150 mTorr, at a temperature of about -55°C to about 40°C. The drying step can be continued in a high temperature range up to room temperature for a predetermined period of up to several days. The level of residual water in the solid lyophilized composition may be less than about 5 w/v%, or less than 4% w/v, or less than 3 w/v%, or less than 2 w/v%, or less than 1% w/v.

### Production method of nucleic acid-encapsulating lipid nanoparticles

The present invention also provides a method for producing nucleic acid-encapsulating lipid nanoparticles by rehydrating the lyophilized composition, obtained by the above-mentioned step a) to step d), in the below-mentioned step e).

### e) step of mixing lyophilized composition with water and optionally incubating the mixture at 0 to 95°C for 0 to 60 min to obtain nucleic acid-encapsulating lipid nanoparticles

In step e), nucleic acid-encapsulating lipid nanoparticles are prepared by adding water to the lyophilized composition of nucleic acid-encapsulating lipid nanoparticles of the present invention. Nucleic acid-encapsulating lipid nanoparticles can be prepared by adding water to the lyophilized composition and mixing same by pipetting or vortexing. In addition, alcohol can be added during rehydration in order to increase the nucleic acid encapsulation rate, and methanol, ethanol, n-butanol and t-butanol, preferably ethanol, can be used as the alcohol. The concentration of alcohol in water to be added to the lyophilized composition is 0 to 50 v/v%, preferably 0 to 30 v/v%, more preferably 0 to 25 v/v%. Furthermore, in order to increase the efficiency of nucleic acid encapsulation in the rehydration step, water or further alcohol is added to the lyophilized composition, after which the mixture can be optionally incubated. The incubation conditions are, for example, 0 to 100°C for 0 to 120 min, or 0 to 95°C for 0 to 60 min, preferably 30 to 100°C for 0 to 60 min.

In step e), a suspension of nucleic acid-encapsulating lipid nanoparticles is obtained by mixing the lyophilized product with water. The concentration of nucleic acid in the suspension is not particularly limited as long as it is within a range in which a suspension can be formed by mixing the lyophilized product with water. From the aspect of particle size of the nucleic acid-encapsulating lipid nanoparticles, it is preferably 0.1 to 4000 µg/mL, more preferably 0.5 to 3000 µg/mL, further preferably 12000 µg/mL. In step e), water may be used in an amount that affords the above-mentioned nucleic acid concentration.

The external aqueous phase of the nucleic acid-encapsulating lipid nanoparticles obtained in step e) may be exchanged with a neutral buffer. Examples of the method of exchanging the external aqueous phase with a neutral buffer include methods using dialysis, ultrafiltration or dilution. Examples of the neutral buffer include phosphate buffered saline (PBS), Tris-buffered saline (TBS), Tris-HCl buffer, N-(2-acetamido)iminodiacetic acid (ADA) buffer, 1,4-piperazinediethanesulfonic acid (PIPES) buffer, 1,4-piperazinediethanesulfonic acid sesquisodium salt (PIPES sesquisodium) buffer, N-(2-acetamido)-2-aminoethanesulfonic acid (ACES) buffer, 3(N-morpholino)propanesulfonic acid (MOPS) buffer, N,N-bis(2-hydroxyethyl)-2-aminoethanesulfonic acid (BES) buffer, 2-hydroxy-3-morpholinopropanesulfonic acid (MOPSO) buffer, N-tris(hydroxymethyl)methyl-2-aminoethanesulfonic acid (TES) buffer, N-(2-hydroxyethyl)piperazine-N'-(2-ethanesulfonic acid (HEPES) buffer, 3-[N-tris(hydroxymethyl)methylamino]-2-hydroxypropanesulfonic acid (TAPSO) buffer, piperazine-1,4-bis(2-hydroxypropanesulfonic acid) (POPSO) buffer, 4-(2-hydroxyethyl)piperazine-1-(2-hydroxypropane-3-sulfonic acid) (HEPSO) buffer, and the like. The pH of the neutral buffer is 6 to 8.

### Method for transferring nucleic acid into cell

A nucleic acid can be transferred into a cell in vivo and/or in vitro by contacting the nucleic acid-encapsulating lipid nanoparticles prepared by the method of the present invention with the cell. Therefore, the present invention provides a method for transferring the nucleic acid into the cell in vivo and/or in vitro.

By the method of the present invention, any nucleic acids can be transferred into cells. Examples of the type of nucleic acid include, but are not limited to, DNA, RNA, chimera nucleic acid of RNA, DNA/RNA hybrid, and the like. While any single-stranded to triple-stranded nucleic acid can be used, it is preferably single-stranded or double-stranded. The nucleic acid may be another type of nucleotide which is N-glycoside of purine or pyrimidine base, other oligomer having a non-nucleotide backbone (e.g., commercially available peptide nucleic acid (PNA) etc.), or other oligomer containing a special bond (said oligomer containing a nucleotide having a configuration permitting base pairing or attachment of base, which are found in DNA and RNA), and the like. Furthermore, the nucleic acid may be, for example, a nucleic acid added with known modification, a nucleic acid with a label known in the field, a nucleic acid with a cap, a methylated nucleic acid, a nucleic acid with one or more natural nucleotides substituted by an analog, a nucleic acid with intramolecular modified nucleotide, a nucleic acid having a non-charge bond (e.g., methylphosphonate, phosphotriester, phosphoramidate, carbamate, and the like), a nucleic acid having a charged bond or sulfur-containing bond (e.g., phosphorothioate, phosphorodithioate, and the like), a nucleic acid having a side chain group such as protein (e.g., nuclease, nuclease inhibitor, toxin, antibody, signal peptide, poly-L-lysine, and the like), sugar (e.g., monosaccharide and the like), and the like, a nucleic acid containing an intercalating compound (e.g., acridine, psoralen, and the like), a nucleic acid containing a chelate compound (e.g., metal, radioactive metal, boron, oxidative metal, and the like), a nucleic acid containing an alkylating agent, or a nucleic acid containing a modified bond (e.g., α anomer-type nucleic acid and the like).

The type of the DNA that can be used in the present invention is not particularly limited, and can be selected as appropriate according to the purpose of use. Examples include plasmid DNA, cDNA, antisense DNA, chromosomal DNA, PAC, BAC, CpG-oligo, and the like. Preferred are plasmid DNA, cDNA and antisense DNA, and more preferred is plasmid DNA. A circular DNA such as plasmid DNA and the like can be digested as appropriate with a restriction enzyme and the like, and also used as a linear DNA.

The type of the RNA that can be used in the present invention is not particularly limited, and can be selected as appropriate according to the purpose of use. Examples thereof include siRNA, miRNA, shRNA, antisense RNA, messenger RNA (mRNA), single-stranded RNA genome, double-stranded RNA genome, RNA replicon, transfer RNA, ribosomal RNA, and the like. Preferred are siRNA, miRNA, shRNA, mRNA, antisense RNA, and RNA replicon.

The nucleic acid used in the present invention is preferably purified by a method generally used by those of ordinary skill in the art.

The nucleic acid-encapsulating lipid nanoparticles produced by the method of the present invention can be administered in vivo for the purpose of, for example, prevention and/or treatment of diseases. Therefore, the nucleic acid to be used in the present invention is preferably one having a preventive and/or therapeutic activity against a given disease (prophylactic/therapeutic nucleic acid). Examples of such nucleic acid include nucleic acids used for so-called gene therapy, and the like.

The nucleic acid-encapsulating lipid nanoparticles produced by the method of the present invention can be used as a drug delivery system for selectively delivering a nucleic acid and the like into a particular cell, and is useful for, for example, DNA vaccines by transferring antigen gene into dendritic cells, gene therapy drugs for tumor, nucleic acid pharmaceutical products that suppress expression of target genes by utilizing RNA interference, and the like. Therefore, the present invention provides a production method of a pharmaceutical composition.

The particle size of the lipid nanoparticles encapsulating a nucleic acid is not particularly limited, and is preferably 10 nm to 500 nm, more preferably 30 nm to 300 nm. The particle size can be measured by using a particle size distribution measuring device such as Zetasizer Nano (Malvern) or the like. The particle size of the lipid nanoparticles can be appropriately adjusted by the method for producing the lipid nanoparticles.

The surface charge (zeta potential) of the lipid nanoparticles encapsulating the nucleic acid is not particularly limited and preferably -15 to +15 mV, more preferably -10 to +10 mV. In conventional transgene, particles electrically charged to have a plus surface potential have been mainly used. This is useful as a method for promoting electrostatic interactions with heparin sulfate on the negatively-charged cell surface to enhance uptake into cells. However, the positive surface charge may suppress, in the cell, release of nucleic acid from the carrier due to the interaction with a nucleic acid to be delivered or protein synthesis due to the interaction between mRNA and a nucleic acid to be delivered. This problem can be solved by adjusting the surface charge to fall within the above-mentioned range. The surface charge can be measured using a zeta potential measuring apparatus such as Zetasizer Nano and the like. The surface charge of the lipid nanoparticles can be adjusted by the formulation of the constituent component of the lipid nanoparticles.

The step of contacting the lipid nanoparticles encapsulating the nucleic acid with the cell in vitro is specifically explained below.

The cells are suspended in a suitable medium several days before contact with the lipid nanoparticles, and cultured under appropriate conditions. At the time of contact with the lipid nanoparticles, the cells may or may not be in a proliferative phase.

The culture medium on contact may be a serum-containing medium or a serum-free medium, wherein the serum concentration of the medium is preferably 30 wt% or less, more preferably 20 wt% or less, since when the medium contains excess protein such as serum and the like, the contact between the lipid nanoparticles and the cell may be inhibited.

The cell density on contact is not particularly limited, and can be appropriately determined in consideration of the kind of the cell and the like. It is generally within the range of 1×10⁴ to 1×10⁷ cells/mL.

A suspension of the aforementioned lipid nanoparticles encapsulating the nucleic acid is added to the thus-prepared cells. The amount of the suspension to be added is not particularly limited, and can be appropriately determined in consideration of the cell number and the like. The concentration of the lipid nanoparticles to be contacted with the cells is not particularly limited as long as the desired transfer of the nucleic acid into the cells can be achieved. The lipid concentration is generally 1 to 300 nmol/ml, preferably 10 to 200 nmol/ml, and the concentration of the nucleic acid is generally 0.01 to 100 ug/ml, preferably 0.05 to 10 µg/ml.

After the aforementioned suspension is added to cells, the cells are cultured. The temperature, humidity and CO₂ concentration during culturing are appropriately determined in consideration of the kind of the cell. When the cell is derived from a mammal, generally, the temperature is about 37°C, humidity is about 95% and CO₂ concentration is about 5%. While the culture period can also be appropriately determined in consideration of the conditions such as the kind of the cell and the like, it is generally a range of 0.1 to 96 hr, preferably a range of 0.2 to 72 hr, more preferably a range of 0.5 to 48 hr. When the above-mentioned culture time is too short, the nucleic acid is not sufficiently transferred into the cells, and when the culture time is too long, the cells may become weak.

By the above-mentioned culture, the nucleic acid is transferred into cells. The culture is further continued preferably by exchanging the medium with a fresh medium, or adding a fresh medium to the medium. When the cell is a mammal-derived cell, the fresh medium preferably contains a serum or nutrition factor.

As mentioned above, a nucleic acid can be transferred into cells not only outside the body (in vitro) but also in the body (in vivo) by using lipid nanoparticles encapsulating the nucleic acid. That is, by administration of the lipid nanoparticles encapsulating the nucleic acid to a subject, the lipid nanoparticles reaches and contacts with the target cells, and the nucleic acid encapsulated in the lipid nanoparticles is transferred into the cells in vivo. The subject to which the lipid nanoparticles can be administered is not particularly limited and, for example, vertebrates such as mammals (e.g., human, monkey, mouse, rat, hamster, bovine, etc.), birds (e.g., chicken, ostrich, etc.), amphibia (e.g., frog etc.), fishes (e.g., zebrafish, rice-fish, etc.), and the like, invertebrates such as insects (e.g., silk moth, moth, Drosophila, etc.) and the like, plants and the like can be mentioned. The subject of administration of the lipid nanoparticles encapsulating the nucleic acid is preferably human or other mammal.

The kind of the target cell is not particularly limited, and a nucleic acid can be transferred into cells in various tissues (e.g., liver, kidney, pancreas, lung, spleen, heart, blood, muscle, bone, brain, stomach, small intestine, large intestine, skin, adipose tissue, lymph node, tumor, etc.) by using the lipid nanoparticles encapsulating the nucleic acid.

The administration method of the lipid nanoparticles into which a nucleic acid and/or a compound other than nucleic acid is introduced to a target (e.g., vertebrate, invertebrate, and the like) is not particularly limited as long as the lipid nanoparticles reaches and contacts with the target cells, and the compound introduced into the lipid nanoparticles can be transferred into the cell, and an administration method known per se (e.g., oral administration, parenteral administration (e.g., intravenous administration, intramuscular administration, topical administration, transdermal administration, subcutaneous administration, intraperitoneal administration, spray, etc.), etc.) can be appropriately selected in consideration of the kind of the compound to be transferred, the kind and the site of the target cell and the like. The dose of the lipid nanoparticles is not particularly limited as long as the transfer of the compound into the cells can be achieved, and can be appropriately selected in consideration of the kind of the subject of administration, administration method, the kind of the compound to be transferred, the kind and the site of the target cell and the like.

When the lipid nanoparticles encapsulating the nucleic acid are used as a nucleic acid transfer agent, they can be formulated according to a conventional method.

When the nucleic acid transfer agent is provided as a reagent for studies, the lipid nanoparticles encapsulating the nucleic acid may be provided as it is as the nucleic acid transfer agent of the present invention, or the nucleic acid transfer agent of the present invention may be provided as a sterile solution or suspension with, for example, water or other physiologically acceptable liquid (e.g., water-soluble solvent (e.g., malic acid buffer, etc.), organic solvent (e.g., ethanol, methanol, DMSO, tert-butanol, and the like), or a mixture of aqueous solvent and organic solvent, etc.). The nucleic acid transfer agent of the present invention may appropriately contain physiologically acceptable additive (e.g., excipient, vehicle, preservative, stabilizer, binder, etc.), which are known per se.

When the nucleic acid transfer agent is provided as a medicament, the lipid nanoparticle encapsulating the nucleic acid may be used as it is as the nucleic acid transfer agent of the present invention or the nucleic acid transfer agent of the present invention may be produced as an oral preparation (for example, tablet, capsule, etc.) or a parenteral agent (for example, injection, spray, etc.), preferably a parenteral agent (more preferably, injection) by blending with a pharmaceutically acceptable known additives such as carrier, flavor, excipient, vehicle, preservative, stabilizer, binder, and the like in a conventionally-admitted unit dosage form required for practicing preparation formulation.

The nucleic acid transfer agent of the present invention can be formulated into a preparation not only for adults but also for children.

### [Example]

The Examples of the present invention are explained in further detail in the following; however, the present invention is not limited in any way by the Examples.

In the following Examples and the like, ionic lipids represented by the formula (1) are shown by the names listed in the aforementioned Tables. Nucleic acid-encapsulating lipid nanoparticles are sometimes referred to as "nucleic acid-encapsulating nanoparticles". The abbreviations used in the following Examples and the like each mean the following.
Chol: cholesterol
DMG-PEG2000: 1,2-dimyristoyl-rac-glycerol, methoxypolyethylene glycol (number average molecular weight (Mn) of PEG: 2000)
DOPC: 1,2-dioleoyl-sn-glycero-3-phosphocholine
MES: 2-morpholinoethanesulfonic acid
PBS: phosphate buffered saline
TBS: tris buffered saline
DDW: deionized distilled water
tBuOH: tert-butanol
EtOH: ethanol

### Preparation method of lyophilized composition of nucleic acid-encapsulating lipid nanoparticles of Comparative Example 1

SS-OP was used as the ionic lipid (1), and DOPC, Chol, and DMG-PEG2000 were used as other lipids. The molar ratio of SS-OP:DOPC:Chol used was 52.5:7.5:40, and 1.5 mol% of DMG-PEG2000 was used with respect to the total of SS-OP, DOPC, and Chol. They were mixed to a total lipid of 10 mM.

An aqueous solution (2840 µL) of mRNA (CleanCap (registered trademark) FLuc mRNA (L-7602), hereafter referred to as "FLuc mRNA") diluted with an acidic malic acid buffer (buffer concentration 20 mM, pH 3.0) to an mRNA concentration of 12.5 µg/mL, and a lipid tert-butanol solution (710 µL) were each weighed into syringes. Using NanoAssmblr (registered trademark) ultra-high-speed nanomedicine production device (manufactured by Precision NanoSystems), LNP was prepared under the conditions of nucleic acid solution:lipid tert-butanol solution flow ratio of 4:1, total flow rate of 1 mL/min, and syringe holder temperature: 25°C. A sucrose solution was added to the recovered LNP to a final concentration of 160 mg/mL. The obtained mixture was placed in screw tubes by 600 µL and lyophilized to obtain a lyophilized composition (ionic lipid (1) content: 0.77 wt%, nucleic acid content: 6 µg, N/P ratio: 67.5, sucrose content: 99.9 wt%).

For lyophilizing, the solution was first frozen at normal pressure at -55°C for 14 to 21 hr, then the pressure was reduced to 200 mTorr (26Pa) and the temperature was raised by 10°C. The temperature rise program was set to raise the temperature by 10°C over 3 hr up to -20°C, raise the temperature by 10°C over 2 hr above -10°C, and allow the same to stand at said temperature for 1 hr. When the temperature rose to 30°C, the same was allowed to stand at said temperature for 3 hr, the pressure was returned to normal pressure, and the lyophilized composition was collected.

DDW (300 µL) was added to the lyophilized composition and stirred using a vortex mixer to obtain a suspension of nucleic acid-encapsulating nanoparticles (nucleic acid concentration: 20 µg/mL).

### Preparation method of lyophilized composition of nucleic acid-encapsulating lipid nanoparticles of Example 1

SS-OP was used as the ionic lipid (1), and DOPC, Chol, and DMG-PEG2000 were used as other lipids. The molar ratio of SS-OP:DOPC:Chol used was 52.5:7.5:40, and 1.5 mol% of DMG-PEG2000 was used with respect to the total of SS-OP, DOPC, and Chol. They were mixed to a total lipid of 7.25 mM.

A solution (2919 µL) of FLuc mRNA diluted with an acidic malic acid buffer (buffer concentration 20 mM, pH 3.0) to 12.1 µg/mL, and a lipid ethanol solution (973 µL) were each weighed into syringes. Using NanoAssmblr (registered trademark) ultra-high-speed nanomedicine production device (manufactured by Precision NanoSystems), LNP was prepared under the conditions of nucleic acid solution:lipid ethanol solution flow ratio of 3:1, total flow rate of 4 mL/min, and syringe holder temperature: 25°C. After adding 4000 µL of MES buffer (buffer concentration 20 mM, pH 6.0) to the recovered material, the obtained mixture was transferred to Amicon Ultra 15 and concentrated to about 2000 µL by ultrafiltration under centrifugation conditions (25°C, 1000 g, 5 min). The obtained concentrate was diluted to 15 mL with MES buffer (buffer concentration 20 mM, pH 6.0) and again concentrated to about 1500 µL by ultrafiltration under centrifugation conditions (25°C, 1000 g, 5 min). The obtained concentrate was diluted to 15 mL with MES buffer (buffer concentration 20 mM, pH 6.0), again concentrated to about 1000 µL by ultrafiltration under centrifugation conditions (25°C, 1000 g, 5 min), and diluted with MES buffer (buffer concentration 20 mM, pH 6.0). A mixture containing nucleic acid-encapsulating lipid nanoparticles (3300 µL, nucleic acid concentration 10 µg/mL) was recovered, and a sucrose solution was added to a final concentration of 160 mg/mL. The obtained mixture was placed in screw tubes by 600 µL and lyophilized to obtain a lyophilized composition (ionic lipid (1) content: 0.77 wt%, nucleic acid content: 6 µg, N/P ratio: 67.5, sucrose content: 99.9 wt%).

For lyophilizing, the solution was first frozen at normal pressure at -55°C for 14 to for 21 hr, then the pressure was reduced to 200 mTorr (26Pa) and the temperature was raised by 10°C. The temperature rise program was set to raise the temperature by 10°C over for 3 hr up to -20°C, raise the temperature by 10°C over for 2 hr above -10°C, and allow the same to stand at said temperature for 1 hr. When the temperature rose to 30°C, the same was allowed to stand at said temperature for 3 hr, the pressure was returned to normal pressure, and the lyophilized composition was collected.

DDW (600 µL) was added to the lyophilized composition and stirred using a vortex mixer to obtain a suspension of nucleic acid-encapsulating nanoparticles (nucleic acid concentration: 10 µg/mL).

### Preparation method of lyophilized composition of nucleic acid-encapsulating lipid nanoparticles of Comparative Example 2

SS-OP was used as the ionic lipid (1), and DOPC, Chol, and DMG-PEG2000 were used as other lipids. The molar ratio of SS-OP:DOPC:Chol used was 52.5:7.5:40, and 1.5 mol% of DMG-PEG2000 was used with respect to the total of SS-OP, DOPC, and Chol. They were mixed to a total lipid of 7.25 mM.

A solution (2919 µL) of FLuc mRNA diluted with an acidic malic acid buffer (buffer concentration 20 mM, pH 3.0) to 12.1 µg/mL, and a lipid ethanol solution (973 µL) were each weighed into syringes. Using NanoAssmblr (registered trademark) ultra-high-speed nanomedicine production device (manufactured by Precision NanoSystems), LNP was prepared under the conditions of nucleic acid solution:lipid ethanol solution flow ratio of 3:1, total flow rate of 4 mL/min, and syringe holder temperature: 25°C. After adding 4000 µL of MES buffer (buffer concentration 20 mM, pH 6.5) to the recovered material, the obtained mixture was transferred to Amicon Ultra 15 and concentrated to about 2000 µL by ultrafiltration under centrifugation conditions (25°C, 1000 g, 5 min). The obtained concentrate was diluted to 15 mL with PBS (pH 7.4) and again concentrated to about 1500 µL by ultrafiltration under centrifugation conditions (25°C, 1000 g, 5 min). The obtained concentrate was diluted to 15 mL with PBS (pH 7.4), again concentrated to about 1000 µL by ultrafiltration under centrifugation conditions (25°C, 1000 g, 5 min), and diluted with PBS (pH 7.4). A mixture containing nucleic acid-encapsulating lipid nanoparticles (3300 µL, nucleic acid concentration 10 µg/mL) was recovered, and a sucrose solution was added to a final concentration of 160 mg/mL. The obtained mixture was placed in screw tubes by 600 µL and lyophilized to obtain a lyophilized composition (ionic lipid (1) content: 0.77 wt%, nucleic acid content: 6 µg, N/P ratio: 67.5, sucrose content: 99.9 wt%).

For lyophilizing, the solution was first frozen at normal pressure at -55°C for 14 to 21 hr, then the pressure was reduced to 200 mTorr (26Pa) and the temperature was raised by 10°C. The temperature rise program was set to raise the temperature by 10°C over 3 hr up to -20°C, raise the temperature by 10°C over 2 hr above -10°C, and allow the same to stand at said temperature for 1 hr. When the temperature rose to 30°C, the same was allowed to stand at said temperature for 3 hr, the pressure was returned to normal pressure, and the lyophilized composition was collected.

DDW (600 µL) was added to the lyophilized composition and stirred using a vortex mixer to obtain a suspension of nucleic acid-encapsulating nanoparticles (nucleic acid concentration: 10 µg/mL).

### Preparation method of lipid nanoparticles of Comparative

### Example 3

SS-OP was used as the ionic lipid (1), and DOPC, Chol, and DMG-PEG2000 were used as other lipids. The molar ratio of SS-OP:DOPC:Chol used was 52.5:7.5:40, and 1.5 mol% of DMG-PEG2000 was used with respect to the total of SS-OP, DOPC, and Chol. They were mixed to a total lipid of 3.0 mM.

A solution (788 µL) of FLuc mRNA diluted with an acidic malic acid buffer (buffer concentration 20 mM, pH 3.0) to 5 µg/mL, and a lipid ethanol solution (263 µL) were each weighed into syringes. Using NanoAssmblr (registered trademark) ultra-high-speed nanomedicine production device (manufactured by Precision NanoSystems), LNP was prepared under the conditions of nucleic acid solution:lipid ethanol solution flow ratio of 3:1, total flow rate of 4 mL/min, and syringe holder temperature: 25°C. After adding 1000 µL of MES buffer (buffer concentration 20 mM, pH 6.5) to the recovered material, the obtained mixture was transferred to Amicon Ultra 15 and concentrated to about 500 µL by ultrafiltration under centrifugation conditions (25°C, 1000 g, 5 min). The obtained concentrate was diluted to 15 mL with PBS (pH 7.4) and again concentrated to about 500 µL by ultrafiltration under centrifugation conditions (25°C, 1000 g, 5 min). The above operation was repeated twice, and the suspension was concentrated to 300 µL to obtain a suspension of nucleic acid-encapsulating nanoparticles (nucleic acid concentration: 10 µg/mL).

The particle size, polydispersity index (PdI), and mRNA encapsulation rate of the obtained nucleic acid-encapsulated nanoparticles were analyzed. The particle size and PdI were measured by a dynamic light scattering method using Zetasizer (registered trademark). The mRNA encapsulation rate was measured by Ribogreen (registered trademark) assay. The pH of the suspension of the nucleic acid-encapsulating nanoparticles after thawing the lyophilized composition with DDW in Comparative Examples 1 and 2 and Example 1 (hereinafter referred to as "pH after thawing") was measured with a pH meter (Table 2). The particle size is shown as Z-Ave (Z-average) and Number Mean (number average) (hereinafter the same).

The nucleic acid-encapsulating nanoparticles obtained were applied to HeLa cells to evaluate the nucleic acid transfer efficiency. HeLa cells were seeded in D-MEM medium (high glucose) (containing L-glutamine and phenol red) (containing 10% FBS by volume and 1% penicillin-streptomycin by volume) at 1.5×10⁴ cells/300 µL into a 48-well flat-bottom transparent plate. The obtained suspension of nucleic acid-encapsulated nanoparticles was added such that the nucleic acid concentration in the medium was 0.06 ug/30 µL. At 24 hr from the addition, 300 µL of Steady-Glo (registered trademark) Luciferase assay system was added, and the mixture was incubated for 5 min while shielded from light with aluminum foil, and the luminescence at 560 nm was measured with a plate reader. As a result, among the nucleic acid-encapsulating nanoparticles produced by the freeze-drying technique, the nucleic acid-encapsulating nanoparticles produced by the freeze-drying technique in Example 1 had the highest gene expression activity, and showed the same activity as the nucleic acid-encapsulating nanoparticles produced by the method of Comparative Example 3 without freeze-drying (see Fig. 1).

**[Table 2]**

| | presence or absence of lyophilizing step | solvent | pH after thawing | encapsulation rate (%) | Z-Ave (d.nm) | Number Mean (d.nm) | PdI |
|---|---|---|---|---|---|---|---|
| Comp. Ex. 1 | present | tBuOH | 3.2 | 24 | 324 | 251 | 0.18 |
| Ex. 1 | | EtOH | 6.6 | 56 | 270 | 208 | 0.16 |
| Comp. Ex. 2 | | EtOH | 7.2 | 45 | 209 | 142 | 0.15 |
| Comp. Ex. 3 | absent | EtOH | - | 91 | 90 | 64 | 0.07 |

### Preparation method of lipid nanoparticles of Comparative Example 4

SS-OP was used as the ionic lipid (1), and DOPC, Chol, and DMG-PEG2000 were used as other lipids. The molar ratio of SS-OP:DOPC:Chol used was 52.5:7.5:40, and 1.5 mol% of DMG-PEG2000 was used with respect to the total of SS-OP, DOPC, and Chol. They were mixed to a total lipid of 4.0 mM.

A solution (1986 µL) of FLuc mRNA diluted with an acidic malic acid buffer (buffer concentration 20 mM, pH 3.0) to 6.7 µg/mL, and a lipid ethanol solution (663 µL) were each weighed into syringes. Using NanoAssmblr (registered trademark) ultra-high-speed nanomedicine production device (manufactured by Precision NanoSystems), LNP was prepared under the conditions of nucleic acid solution:lipid ethanol solution flow ratio of 3:1, total flow rate of 4 mL/min, and syringe holder temperature: 25°C. After adding 10600 µL of MES buffer (buffer concentration 20 mM, pH 6.5) to the recovered material, the obtained mixture was transferred to Amicon Ultra 15 and concentrated to about 2000 µL by ultrafiltration under centrifugation conditions (25°C, 1000 g, 5 min). The obtained concentrate was diluted to 15 mL with PBS (pH 7.4) and again concentrated to about 2000 µL by ultrafiltration under centrifugation conditions (25°C, 1000 g, 5 min). The above operation was repeated twice, and the suspension was concentrated to 600 µL to obtain a suspension of nucleic acid-encapsulating nanoparticles (nucleic acid concentration: 10 µg/mL).

The particle size, polydispersity index (PdI), and mRNA encapsulation rate of the obtained suspension of nucleic acid-encapsulating nanoparticles were analyzed on the day of preparation, and one week and two weeks after preparation. The particle size and PdI were measured by a dynamic light scattering method using Zetasizer (registered trademark). The mRNA encapsulation rate was measured by Ribogreen (registered trademark) assay. As a result, no change was observed in the particle properties, and the particle size and encapsulation rate were maintained for two weeks after preparation, indicating high storage stability (see Table 3).

**[Table 3]**

| | time point | encapsulation rate (%) | Z-Ave (d.nm) | Number Mean (d.nm) | PdI |
|---|---|---|---|---|---|
| Comparative Example 4 | Day 0 | 81 | 94 | 65 | 0.10 |
| | week 1 | 88 | 95 | 69 | 0.08 |
| | week 2 | 85 | 94 | 64 | 0.09 |

The nucleic acid-encapsulating nanoparticles obtained were applied to HeLa cells to evaluate the nucleic acid transfer efficiency. HeLa cells were seeded in D-MEM medium (high glucose) (containing L-glutamine and phenol red) (containing 10% FBS by volume and 1% penicillin-streptomycin by volume) at 5×10³ cells/100 µL into a 35 mm dish. The obtained suspension of nucleic acid-encapsulated nanoparticles was added such that the nucleic acid concentration in the medium was 0.4 µg/200 µL. Luciferin was added to the medium to 0.1 mM, and the amount of luciferase protein introduced was evaluated over time using the amount of luminescence. HeLa cells cultured in a medium containing nucleic acid-encapsulating nanoparticles and luciferin were placed in an incubator-type luminometer (Kronos), and the cumulative amount of luminescence was measured for 2 minutes every hour. As a result, a decrease in gene expression activity was observed from immediately after preparation until the first week, and the activity further decreased in the second week, indicating low storage stability (see Fig. 2). In Fig. 2, "1.4.E+07", etc. mean "1.4×10⁷" and the like.

### Preparation method of lyophilized composition of nucleic acid-encapsulating lipid nanoparticles of Example 2

SS-OP was used as the ionic lipid (1), and DOPC, Chol, and DMG-PEG2000 were used as other lipids. The molar ratio of SS-OP:DOPC:Chol used was 52.5:7.5:40, and 1.5 mol% of DMG-PEG2000 was used with respect to the total of SS-OP, DOPC, and Chol. They were mixed to a total lipid of 16.0 mM.

An acidic malic acid buffer (2918 µL) containing mRNA encoding human erythropoietin (CleanCap (registered trade mark) hEPO mRNA (L-7209)) (buffer concentration 20 mM, pH 3.0, nucleic acid concentration 26.7 µg/mL), and a lipid ethanol solution (973 µL) were each weighed into syringes. Using NanoAssmblr (registered trademark) ultra-high-speed nanomedicine production device (manufactured by Precision NanoSystems), LNP was prepared under the conditions of nucleic acid solution:lipid ethanol solution flow ratio of 3:1, total flow rate of 4 mL/min, and syringe holder temperature: 25°C. After adding 8400 µL of MES buffer (buffer concentration 20 mM, pH 6.0) to the recovered material, the obtained mixture was divided into 5 equal portions into Amicon Ultra 15. It was diluted to 15 mL with MES buffer (buffer concentration 20 mM, pH 6.0) and concentrated to about 1500 µL by ultrafiltration under centrifugation conditions (25°C, 1000 g, 5 min). The obtained concentrate was diluted to 15 mL with MES buffer (buffer concentration 20 mM, pH 6.0), again concentrated to about 1500 µL by two times of ultrafiltration under centrifugation conditions (25°C, 1000 g, 5 min) to obtain a mixture containing nucleic acid-encapsulating lipid nanoparticles (nucleic acid concentration 10 µg/mL). A sucrose solution was added to a final concentration of 160 mg/mL. The obtained mixture was placed in screw tubes by 600 µL and lyophilized to obtain a lyophilized composition (ionic lipid (1) content: 0.76 wt%, nucleic acid content: 6 µg, N/P ratio: 67.5, sucrose content: 99.9 wt%). The obtained lyophilized composition was stored at -20°C, 4°C, or 25°C.

For lyophilizing, the solution was first frozen at normal pressure at -40°C for 150 min, then the pressure was reduced to 80 mTorr (11Pa) and the same was left standing for 390 min. Thereafter, the temperature was raised by 10°C. The temperature rise program was set to raise the temperature by 10°C over 10 min up to 0°C, and allow the same to stand at said temperature for 6 hr. At 0°C or above, the temperature was raised by 10°C over 10 min, and the same was left standing at said temperature for 3 hr. When the temperature rose to 30°C, the same was allowed to stand at said temperature for 3 hr, the pressure was returned to normal pressure, and the lyophilized composition was collected.

The lyophilized composition was rehydrated by adding 600 µL of DDW and stirring using a vortex mixer. To investigate the influence of incubation after rehydration, a portion of the composition was incubated at 95°C for 5 min using an aluminum block incubator after rehydration. After the incubation suspension was cooled with water at 20°C to 25°C, 600 µL of PBS (pH 7.4) was added to obtain a suspension of nucleic acid-encapsulating nanoparticles (nucleic acid concentration: 5 µg/mL).

### Preparation of lipid nanoparticles of Comparative Example 4

SS-OP was used as the ionic lipid (1), and DOPC, Chol, and DMG-PEG2000 were used as other lipids. The molar ratio of SS-OP:DOPC:Chol used was 52.5:7.5:40, and 1.5 mol% of DMG-PEG2000 was used with respect to the total of SS-OP, DOPC, and Chol.

An acidic malic acid buffer (2288 µL) containing mRNA encoding human erythropoietin (CleanCap (registered trade mark) hEPO mRNA (L-7209)) (buffer concentration 20 mM, pH 3.0, nucleic acid concentration 6.7 µg/mL), and a lipid ethanol solution (863 µL) were each weighed into syringes. Using NanoAssmblr (registered trademark) ultra-high-speed nanomedicine production device (manufactured by Precision NanoSystems), LNP was prepared under the conditions of nucleic acid solution:lipid ethanol solution flow ratio of 3:1, total flow rate of 4 mL/min, and syringe holder temperature: 25°C. After adding 2400 µL of MES buffer (buffer concentration 20 mM, pH 6.5) to the recovered material, the obtained mixture was transferred to Amicon Ultra 15 and concentrated to about 2000 µL by ultrafiltration under centrifugation conditions (25°C, 1000 g, 5 min). The obtained concentrate was diluted to 15 mL with PBS (pH 7.4) and again concentrated to about 1000 µL by ultrafiltration under centrifugation conditions (25°C, 1000 g, 5 min). The above operation was repeated twice, and the suspension was concentrated to 800 µL to obtain a suspension of nucleic acid-encapsulating nanoparticles (nucleic acid concentration: 10 µg/mL).

The particle size, polydispersity index (PdI), and mRNA encapsulation rate of the obtained nucleic acid-encapsulating nanoparticles of Example 2 were analyzed on each time point of Day 0, 2 weeks, 1 month, and 3 months. The particle size and PdI were measured by a dynamic light scattering method using Zetasizer (registered trademark). The mRNA encapsulation rate was measured by Ribogreen (registered trademark) assay. As a result, no change was observed in the particle properties at each storage temperature during storage for 3 months, and the particle properties were maintained, indicating high storage stability (see Table 4).

**[Table 4]**

| | storage temperature (°C) | incubation temperature (°C) | time point | encapsulation rate (%) | Z-Ave (d.nm) | Number Mean (d.nm) | PdI |
|---|---|---|---|---|---|---|---|
| 1 | -20 | none | Day 0 | 72 | 135 | 86 | 0.22 |
| 2 | -20 | 95 | | 73 | 151 | 98 | 0.14 |
| 3 | 4 | | | 80 | 144 | 99 | 0.14 |
| 4 | 25 | | | 82 | 141 | 97 | 0.13 |
| 5 | -20 | none | 2 weeks | 76 | 134 | 80 | 0.26 |
| 6 | -20 | 95 | | 78 | 153 | 102 | 0.16 |
| 7 | 4 | | | 80 | 152 | 108 | 0.12 |
| 8 | 25 | | | 76 | 149 | 96 | 0.16 |
| 9 | -20 | none | 1 month | 72 | 149 | 85 | 0.20 |
| 10 | -20 | 95 | | 79 | 147 | 103 | 0.14 |
| 11 | 4 | | | 78 | 154 | 112 | 0.13 |
| 12 | 25 | | | 81 | 152 | 111 | 0.12 |
| 13 | -20 | none | 3 months | 72 | 138 | 81 | 0.19 |
| 14 | -20 | 95 | | 69 | 154 | 98 | 0.15 |
| 15 | 4 | | | 75 | 150 | 100 | 0.18 |
| 16 | 25 | | | 82 | 147 | 92 | 0.15 |

The obtained suspension of nucleic acid-encapsulating nanoparticles was administered to the tail vein of a mouse (Balb/c, 6 weeks old, female). At 6 hours and 24 hours from administration, 15 µL of blood was collected from the mouse tail vein, and the collected blood was immediately mixed with 0.3 µL of heparin solution (5000 U/5 mL). Each blood sample was centrifuged under centrifugation conditions (25°C, 2000 g, 20 min), and the supernatant was recovered. The concentration of erythropoietin in the supernatant was measured using Mouse Erythropoietin Quantikine ELISA Kit (manufactured by R&D Systems) and by the method described in the protocol of the Kit. As a result, it was demonstrated that the gene expression activity was maintained even after storage at 25°C for 3 months, and that the storage stability was high (see Fig. 3).

### Preparation method of lyophilized composition of nucleic acid-encapsulating lipid nanoparticles of Example 3

SS-OP and SS-EC were used as the ionic lipid (1), and DOPC, Chol, and DMG-PEG2000 were used as other lipids. The molar ratio of SS-OP:SS-EC:DOPC:Chol used was 42.5:10:7.5:40, and 1.5 mol% of DMG-PEG2000 was used with respect to the total of SS-OP, SS-EC, DOPC, and Chol. They were mixed to a total lipid of 16 mM.

An acidic malic acid buffer (1688 µL) containing mRNA encoding luciferase (CleanCap (registered trade mark) FLuc mRNA (L-7602)) (buffer concentration 20 mM, pH 3.0, nucleic acid concentration 6.7 µg/mL), and a lipid ethanol solution (563 µL) were each weighed into syringes. Using NanoAssmblr (registered trademark) ultra-high-speed nanomedicine production device (manufactured by Precision NanoSystems), LNP was prepared under the conditions of nucleic acid solution:lipid ethanol solution flow ratio of 3:1, total flow rate of 4 mL/min, and syringe holder temperature: 25°C. After adding 2000 µL of MES buffer (buffer concentration 20 mM, pH 6.0) to the recovered material, the obtained mixture was transferred to Amicon Ultra 15. The mixture was diluted to 15 mL with MES buffer (buffer concentration 20 mM, pH 6.0), and concentrated to about 1500 µL by ultrafiltration under centrifugation conditions (25°C, 1000 g, 5 min). The concentrate was diluted to 15 mL with MES buffer (buffer concentration 20 mM, pH 6.0), and concentrated to about 1500 µL by ultrafiltration twice under centrifugation conditions (25°C, 1000 g, 5 min) to obtain a mixture containing nucleic acid-encapsulating lipid nanoparticles (nucleic acid concentration 10 µg/mL). A sucrose solution was added to a final concentration of 160 mg/mL. The obtained mixture was placed in screw tubes by 600 µL and lyophilized to obtain a lyophilized composition (ionic lipid (1) content: 0.80 wt%, nucleic acid content: 6 µg, N/P ratio: 67.5, sucrose content: 99.9 wt%).

For lyophilizing, the solution was first frozen at normal pressure at -40°C for 150 min, then the pressure was reduced to 80 mTorr (11Pa) and the same was allowed to stand for 390 min. Thereafter, the temperature was raised by 10°C. The temperature rise program was set to raise the temperature by 10°C over 10 min up to 0°C, and allow the same to stand at said temperature for 6 hr. When above 0°C, the temperature was raised by 10°C over 10 min, and the same was allowed to stand at said temperature for 3 hr. When the temperature rose to 30°C, the same was allowed to stand at said temperature for 3 hr, the pressure was returned to normal pressure, and the lyophilized composition was collected.

DDW was added to the lyophilized composition and stirred using a vortex mixer to thaw the lyophilized composition. The obtained mixture was incubated at 95°C for 5 min using an aluminum block incubator. After the incubation suspension was cooled with water at 20°C to 25°C, 600 µL of PBS (pH 7.4) was added to obtain a suspension of nucleic acid-encapsulating nanoparticles (nucleic acid concentration: 5 µg/mL).

### Preparation method of lyophilized composition of nucleic acid-encapsulating lipid nanoparticles of Example 4

SS-OP and SS-EC were used as the ionic lipid (1), and DOPC, Chol, and DMG-PEG2000 were used as other lipids. The molar ratio of SS-OP:SS-EC:DOPC:Chol used was 32.5:20:7.5:40, and 1.5 mol% of DMG-PEG2000 was used with respect to the total of SS-OP, SS-EC, DOPC, and Chol. They were mixed to a total lipid of 16 mM.

An acidic malic acid buffer (1688 µL) containing mRNA encoding luciferase (CleanCap (registered trade mark) FLuc mRNA (L-7602)) (buffer concentration 20 mM, pH 3.0, nucleic acid concentration 26.7 µg/mL), and a lipid ethanol solution (563 µL) were each weighed into syringes. Using NanoAssmblr (registered trademark) ultra-high-speed nanomedicine production device (manufactured by Precision NanoSystems), LNP was prepared under the conditions of nucleic acid solution:lipid ethanol solution flow ratio of 3:1, total flow rate of 4 mL/min, and syringe holder temperature: 25°C. After adding 2000 µL of MES buffer (buffer concentration 20 mM, pH 6.0) to the recovered material, the obtained mixture was transferred to Amicon Ultra 15. The mixture was diluted to 15 mL with MES buffer (buffer concentration 20 mM, pH 6.0), and concentrated to about 1500 µL by ultrafiltration under centrifugation conditions (25°C, 1000 g, 5 min). The concentrate was diluted to 15 mL with MES buffer (buffer concentration 20 mM, pH 6.0), and concentrated to about 1500 µL by ultrafiltration twice under centrifugation conditions (25°C, 1000 g, 5 min) to obtain a mixture containing nucleic acid-encapsulating lipid nanoparticles (nucleic acid concentration 10 µg/mL). A sucrose solution was added to a final concentration of 160 mg/mL. The obtained mixture was placed in screw tubes by 600 µL and lyophilized to obtain a lyophilized composition (ionic lipid (1) content: 0.83 wt%, nucleic acid content: 6 µg, N/P ratio: 67.5, sucrose content: 99.9 wt%).

For lyophilizing, the solution was first frozen at normal pressure at -40°C for 150 min, then the pressure was reduced to 80 mTorr (11Pa) and the same was left standing for 390 min. Thereafter, the temperature was raised by 10°C. The temperature rise program was set to raise the temperature by 10°C over 10 min up to 0°C, and allow the same to stand at said temperature for 6 hr. At 0°C or above, the temperature was raised by 10°C over 10 min, and the same was left standing at said temperature for 3 hr. When the temperature rose to 30°C, the same was allowed to stand at said temperature for 3 hr, the pressure was returned to normal pressure, and the lyophilized composition was collected.

DDW was added to the lyophilized composition and stirred using a vortex mixer to thaw the lyophilized composition. The obtained mixture was incubated at 95°C for 5 min using an aluminum block incubator. After the incubation suspension was cooled with water at 20°C to 25°C, 600 µL of PBS was added to obtain a suspension of nucleic acid-encapsulating nanoparticles (nucleic acid concentration: 5 µg/mL).

### Preparation method of lyophilized composition of nucleic acid-encapsulating lipid nanoparticles of Example 5

SS-OP and SS-EC were used as the ionic lipid (1), and DOPC, Chol, and DMG-PEG2000 were used as other lipids. The molar ratio of SS-OP:SS-EC:DOPC:Chol used was 22.5:30:7.5:40, and 1.5 mol% of DMG-PEG2000 was used with respect to the total of SS-OP, SS-EC, DOPC, and Chol. They were mixed to a total lipid of 10.7 mM.

An acidic malic acid buffer (1688 µL) containing mRNA encoding luciferase (CleanCap (registered trade mark) FLuc mRNA (L-7602)) (buffer concentration 20 mM, pH 3.0, nucleic acid concentration 17.8 µg/mL), and a lipid ethanol solution (563 µL) were each weighed into syringes. Using NanoAssmblr (registered trademark) ultra-high-speed nanomedicine production device (manufactured by Precision NanoSystems), LNP was prepared under the conditions of nucleic acid solution:lipid ethanol solution flow ratio of 3:1, total flow rate of 4 mL/min, and syringe holder temperature: 25°C. After adding 2000 µL of MES buffer (buffer concentration 20 mM, pH 6.0) to the recovered material, the obtained mixture was transferred to Amicon Ultra 15. The mixture was diluted to 15 mL with MES buffer (buffer concentration 20 mM, pH 6.0), and concentrated to about 1500 µL by ultrafiltration under centrifugation conditions (25°C, 1000 g, 5 min). The concentrate was diluted to 15 mL with MES buffer (buffer concentration 20 mM, pH 6.0), and concentrated to about 1300 µL by ultrafiltration twice under centrifugation conditions (25°C, 1000 g, 5 min) to obtain a mixture containing nucleic acid-encapsulating lipid nanoparticles (nucleic acid concentration 10 µg/mL). A sucrose solution was added to a final concentration of 160 mg/mL. The obtained mixture was placed in screw tubes by 600 µL and lyophilized to obtain a lyophilized composition (ionic lipid (1) content: 0.86 wt%, nucleic acid content: 6 µg, N/P ratio: 67.5, sucrose content: 99.9 wt%).

For lyophilizing, the solution was first frozen at normal pressure at -40°C for 150 min, then the pressure was reduced to 80 mTorr (11Pa) and the same was left standing for 390 min. Thereafter, the temperature was raised by 10°C. The temperature rise program was set to raise the temperature by 10°C over 10 min up to 0°C, and allow the same to stand at said temperature for 6 hr. At 0°C or above, the temperature was raised by 10°C over 10 min, and the same was left standing at said temperature for 3 hr. When the temperature rose to 30°C, the same was allowed to stand at said temperature for 3 hr, the pressure was returned to normal pressure, and the lyophilized composition was collected.

DDW was added to the lyophilized composition and stirred using a vortex mixer to thaw the lyophilized composition. The mixture was incubated at 95°C for 5 min using an aluminum block incubator. After the incubation suspension was cooled with water at 20°C to 25°C, 600 µL of PBS was added to obtain a suspension of nucleic acid-encapsulating nanoparticles (nucleic acid concentration: 5 µg/mL).

The pH of the suspension of the nucleic acid-encapsulating nanoparticles after thawing each lyophilized composition with DDW (hereinafter referred to as "pH after thawing") was measured. The particle size, polydispersity index (PdI), and mRNA encapsulation rate of the obtained nucleic acid-encapsulated nanoparticles on day 4 and day 21 after preparation were analyzed. The particle size and PdI were measured by a dynamic light scattering method using Zetasizer (registered trademark). The mRNA encapsulation rate was measured by Ribogreen (registered trademark) assay. As a result, no change was observed in the particle properties, and the particle size and encapsulation rate were maintained for 3 weeks after preparation even when the formulation ratio of SS-EC was changed, indicating high storage stability. The particle size and mRNA encapsulation rate were equivalent to those of the nucleic acid-encapsulating nanoparticles prepared using only SS-OP as the ionic lipid (1) (see Table 5).

**[Table 5]**

| | SS-OP (mol%) | SS-EC (mol%) | time point | pH after thawing | encapsulation rate (%) | Z-Ave (d.nm) | Number Mean (d.nm) | PdI |
|---|---|---|---|---|---|---|---|---|
| Example 3 | 42.5 | 10 | Day 4 | 5.9 | 85 | 170 | 120 | 0.12 |
| Example 4 | 32.5 | 20 | | 6.0 | 81 | 200 | 167 | 0.13 |
| Example 5 | 22.5 | 30 | | 6.0 | 78 | 207 | 176 | 0.08 |
| Example 3 | 42.5 | 10 | Day 21 | 6.0 | 85 | 169 | 133 | 0.12 |
| Example 4 | 32.5 | 20 | | 6.0 | 80 | 189 | 145 | 0.13 |
| Example 5 | 22.5 | 30 | | 6.1 | 84 | 182 | 142 | 0.14 |

The nucleic acid-encapsulating nanoparticles obtained were applied to HeLa cells to evaluate the nucleic acid transfer efficiency. HeLa cells were seeded in D-MEM medium (high glucose) (containing L-glutamine and phenol red) (containing 10% FBS by volume and 1% penicillin-streptomycin by volume) at 5×10³ cells/100 µL into a 35 mm dish. The obtained suspension of nucleic acid-encapsulated nanoparticles was added such that the nucleic acid concentration in the medium was 0.4 µg/200 µL. Luciferin was added to the medium to 0.1 mM, and the amount of luciferase protein introduced was evaluated over time using the amount of luminescence. HeLa cells cultured in a medium containing nucleic acid-encapsulating nanoparticles and luciferin were placed in an incubator-type luminometer (Kronos), and the cumulative amount of luminescence was measured for 2 minutes every hour. As a result, the highest gene transfer efficiency was obtained in Example 4, in which the content of SS-EC relative to the total of ionic lipid (1), phospholipid, and sterol was 20 mol%, and the transfer rate thereof was maintained even after storage for 21 days, indicating high storage stability (see Fig. 4). In Fig. 4, "1.2.E+07", etc. mean "1.2×10⁷" and the like.

### Preparation method of lyophilized composition of nucleic acid-encapsulating lipid nanoparticles of Example 6

SS-OP was used as the ionic lipid (1), and DOPC, Chol, and DMG-PEG2000 were used as other lipids. The molar ratio of SS-OP:DOPC:Chol used was 52.5:7.5:40, and 1.5 mol% of DMG-PEG2000 was used with respect to the total of SS-OP, DOPC, and Chol. They were mixed to a total lipid of 16 mM.

An acidic citrate buffer (2325 µL) containing mRNA encoding luciferase (CleanCap (registered trade mark) FLuc mRNA (L-7602)) (buffer concentration 20 mM, pH 5.0, nucleic acid concentration 26.7 µg/mL), and a lipid ethanol solution (775 µL) were each weighed into syringes. Using NanoAssmblr (registered trademark) ultra-high-speed nanomedicine production device (manufactured by Precision NanoSystems), LNP was prepared under the conditions of nucleic acid solution:lipid ethanol solution flow ratio of 3:1, total flow rate of 12 mL/min, and syringe holder temperature: 25°C. After adding 2600 µL of citrate buffer (buffer concentration 20 mM, pH 6.0) to the recovered material, the obtained mixture was transferred to Amicon Ultra 15. The mixture was diluted to 15 mL with citrate buffer (buffer concentration 20 mM, pH 6.0), and concentrated to about 2000 µL by ultrafiltration under centrifugation conditions (25°C, 1000 g, 5 min). The concentrate was diluted to 15 mL with citrate buffer (buffer concentration 20 mM, pH 6.0), and concentrated to about 1300 µL by ultrafiltration twice under centrifugation conditions (25°C, 1000 g, 5 min) to obtain a mixture containing nucleic acid-encapsulating lipid nanoparticles (nucleic acid concentration 10 µg/mL). A sucrose solution was added to a final concentration of 160 mg/mL. The obtained mixture was placed in screw tubes by 600 µL and lyophilized to obtain a lyophilized composition (ionic lipid (1) content: 0.77 wt%, nucleic acid content: 6 µg, N/P ratio: 67.5, sucrose content: 99.9 wt%).

For lyophilizing, the solution was first frozen at normal pressure at -40°C for 150 min, then the pressure was reduced to 80 mTorr (11Pa) and the same was allowed to stand for 360 min. Thereafter, the pressure was reduced to 50 mTorr (6.7 Pa) and the same was left standing for 840 min. Thereafter, the temperature was raised by 10°C. The temperature rise program was set to raise the temperature by 10°C over 10 min up to 0°C, and allow the same to stand at said temperature for 6 hr. When above 0°C, the temperature was raised by 10°C over 10 min, and the same was allowed to stand at said temperature for 3 hr. When the temperature rose to 30°C, the same was allowed to stand at said temperature for 3 hr, the pressure was returned to normal pressure, and the lyophilized composition was collected.

DDW was added to the lyophilized composition and stirred using a vortex mixer to thaw the lyophilized composition. The obtained mixture was incubated at 95°C for 5 min using an aluminum block incubator. After the incubation suspension was cooled with water at 20°C to 25°C, 600 µL of TBS was added to obtain a suspension of nucleic acid-encapsulating nanoparticles (nucleic acid concentration: 5 µg/mL).

### Preparation method of lyophilized composition of nucleic acid-encapsulating lipid nanoparticles of Example 7

SS-OP and SS-EC were used as the ionic lipid (1), and DOPC, Chol, and DMG-PEG2000 were used as other lipids. The molar ratio of SS-OP:SS-EC:DOPC:Chol used was 32.5:20:7.5:40, and 1.5 mol% of DMG-PEG2000 was used with respect to the total of SS-OP, SS-EC, DOPC, and Chol. They were mixed to a total lipid of 16 mM.

An acidic citrate buffer (2325 µL) containing mRNA encoding luciferase (CleanCap (registered trade mark) FLuc mRNA (L-7602)) (buffer concentration 20 mM, pH 5.0, nucleic acid concentration 26.7 µg/mL), and a lipid ethanol solution (775 µL) were each weighed into syringes. Using NanoAssmblr (registered trademark) ultra-high-speed nanomedicine production device (manufactured by Precision NanoSystems), LNP was prepared under the conditions of nucleic acid solution:lipid ethanol solution flow ratio of 3:1, total flow rate of 12 mL/min, and syringe holder temperature: 25°C. After adding 2600 µL of citrate buffer (buffer concentration 20 mM, pH 6.0) to the recovered material, the obtained mixture was transferred to Amicon Ultra 15. The mixture was diluted to 15 mL with citrate buffer (buffer concentration 20 mM, pH 6.0), and concentrated to about 2000 µL by ultrafiltration under centrifugation conditions (25°C, 1000 g, 5 min). The concentrate was diluted to 15 mL with citrate buffer (buffer concentration 20 mM, pH 6.0), and concentrated to about 1300 µL by ultrafiltration twice under centrifugation conditions (25°C, 1000 g, 5 min) to obtain a mixture containing nucleic acid-encapsulating lipid nanoparticles (nucleic acid concentration 10 µg/mL). A sucrose solution was added to a final concentration of 160 mg/mL. The obtained mixture was placed in screw tubes by 600 µL and lyophilized to obtain a lyophilized composition (ionic lipid (1) content: 0.83 wt%, nucleic acid content: 6 µg, N/P ratio: 67.5, sucrose content: 99.9 wt%).

For lyophilizing, the solution was first frozen at normal pressure at -40°C for 150 min, then the pressure was reduced to 80 mTorr (11Pa) and the same was allowed to stand for 360 min. Thereafter, the pressure was reduced to 50 mTorr (6.7 Pa) and the same was left standing for 840 min. Thereafter, the temperature was raised by 10°C. The temperature rise program was set to raise the temperature by 10°C over 10 min up to 0°C, and allow the same to stand at said temperature for 6 hr. When above 0°C, the temperature was raised by 10°C over 10 min, and the same was allowed to stand at said temperature for 3 hr. When the temperature rose to 30°C, the same was allowed to stand at said temperature for 3 hr, the pressure was returned to normal pressure, and the lyophilized composition was collected.

DDW was added to the lyophilized composition and stirred using a vortex mixer to thaw the lyophilized composition. The obtained mixture was incubated at 95°C for 5 min using an aluminum block incubator. After the incubation suspension was cooled with water at 20°C to 25°C, 600 µL of TBS was added to obtain a suspension of nucleic acid-encapsulating nanoparticles (nucleic acid concentration: 5 µg/mL).

### Preparation method of lyophilized composition of nucleic acid-encapsulating lipid nanoparticles of Example 8

PiP9 was used as the ionic lipid (1), and DOPC, Chol, and DMG-PEG2000 were used as other lipids. The molar ratio of PiP9:DOPC:Chol used was 52.5:7.5:40, and 1.5 mol% of DMG-PEG2000 was used with respect to the total of PiP9, DOPC, and Chol. They were mixed to a total lipid of 16 mM.

An acidic citrate buffer (2325 µL) containing mRNA encoding luciferase (CleanCap (registered trade mark) FLuc mRNA (L-7602)) (buffer concentration 20 mM, pH 5.0, nucleic acid concentration 26.7 µg/mL), and a lipid ethanol solution (775 µL) were each weighed into syringes. Using NanoAssmblr (registered trademark) ultra-high-speed nanomedicine production device (manufactured by Precision NanoSystems), LNP was prepared under the conditions of nucleic acid solution:lipid ethanol solution flow ratio of 3:1, total flow rate of 12 mL/min, and syringe holder temperature: 25°C. After adding 2600 µL of citrate buffer (buffer concentration 20 mM, pH 6.0) to the recovered material, the obtained mixture was transferred to Amicon Ultra 15. The mixture was diluted to 15 mL with citrate buffer (buffer concentration 20 mM, pH 6.0), and concentrated to about 2000 µL by ultrafiltration under centrifugation conditions (25°C, 1000 g, 5 min). The concentrate was diluted to 15 mL with citrate buffer (buffer concentration 20 mM, pH 6.0), and concentrated to about 1300 µL by ultrafiltration twice under centrifugation conditions (25°C, 1000 g, 5 min) to obtain a mixture containing nucleic acid-encapsulating lipid nanoparticles (nucleic acid concentration 10 µg/mL). A sucrose solution was added to a final concentration of 160 mg/mL. The obtained mixture was placed in screw tubes by 600 µL and lyophilized to obtain a lyophilized composition (ionic lipid (1) content: 0.86 wt%, nucleic acid content: 6 µg, N/P ratio: 67.5, sucrose content: 99.9 wt%).

For lyophilizing, the solution was first frozen at normal pressure at -40°C for 150 min, then the pressure was reduced to 80 mTorr (11Pa) and the same was allowed to stand for 360 min. Thereafter, the pressure was reduced to 50 mTorr (6.7 Pa) and the same was left standing for 840 min. Thereafter, the temperature was raised by 10°C. The temperature rise program was set to raise the temperature by 10°C over 10 min up to 0°C, and allow the same to stand at said temperature for 6 hr. When above 0°C, the temperature was raised by 10°C over 10 min, and the same was allowed to stand at said temperature for 3 hr. When the temperature rose to 30°C, the same was allowed to stand at said temperature for 3 hr, the pressure was returned to normal pressure, and the lyophilized composition was collected.

DDW was added to the lyophilized composition and stirred using a vortex mixer to thaw the lyophilized composition. The obtained mixture was incubated at 95°C for 5 min using an aluminum block incubator. After the incubation suspension was cooled with water at 20°C to 25°C, 600 µL of TBS was added to obtain a suspension of nucleic acid-encapsulating nanoparticles (nucleic acid concentration: 5 µg/mL).

### Preparation method of lyophilized composition of nucleic acid-encapsulating lipid nanoparticles of Example 9

PiP15 was used as the ionic lipid (1), and DOPC, Chol, and DMG-PEG2000 were used as other lipids. The molar ratio of PiP15:DOPC:Chol used was 52.5:7.5:40, and 1.5 mol% of DMG-PEG2000 was used with respect to the total of PiP15, DOPC, and Chol. They were mixed to a total lipid of 16 mM.

An acidic citrate buffer (2325 µL) containing mRNA encoding luciferase (CleanCap (registered trade mark) FLuc mRNA (L-7602)) (buffer concentration 20 mM, pH 5.0, nucleic acid concentration 26.7 µg/mL), and a lipid ethanol solution (775 µL) were each weighed into syringes. Using NanoAssmblr (registered trademark) ultra-high-speed nanomedicine production device (manufactured by Precision NanoSystems), LNP was prepared under the conditions of nucleic acid solution:lipid ethanol solution flow ratio of 3:1, total flow rate of 12 mL/min, and syringe holder temperature: 25°C. After adding 2600 µL of citrate buffer (buffer concentration 20 mM, pH 6.0) to the recovered material, the obtained mixture was transferred to Amicon Ultra 15. The mixture was diluted to 15 mL with citrate buffer (buffer concentration 20 mM, pH 6.0), and concentrated to about 2000 µL by ultrafiltration under centrifugation conditions (25°C, 1000 g, 5 min). The concentrate was diluted to 15 mL with citrate buffer (buffer concentration 20 mM, pH 6.0), and concentrated to about 1300 µL by ultrafiltration twice under centrifugation conditions (25°C, 1000 g, 5 min) to obtain a mixture containing nucleic acid-encapsulating lipid nanoparticles (nucleic acid concentration 10 µg/mL). A sucrose solution was added to a final concentration of 160 mg/mL. The obtained mixture was placed in screw tubes by 600 µL and lyophilized to obtain a lyophilized composition (ionic lipid (1) content: 0.87 wt%, nucleic acid content: 6 µg, N/P ratio: 67.5, sucrose content: 99.9 wt%).

For lyophilizing, the solution was first frozen at normal pressure at -40°C for 150 min, then the pressure was reduced to 80 mTorr (11Pa) and the same was allowed to stand for 360 min. Thereafter, the pressure was reduced to 50 mTorr (6.7 Pa) and the same was left standing for 840 min. Thereafter, the temperature was raised by 10°C. The temperature rise program was set to raise the temperature by 10°C over 10 min up to 0°C, and allow the same to stand at said temperature for 6 hr. When above 0°C, the temperature was raised by 10°C over 10 min, and the same was allowed to stand at said temperature for 3 hr. When the temperature rose to 30°C, the same was allowed to stand at said temperature for 3 hr, the pressure was returned to normal pressure, and the lyophilized composition was collected.

DDW was added to the lyophilized composition and stirred using a vortex mixer to thaw the lyophilized composition. The obtained mixture was incubated at 95°C for 5 min using an aluminum block incubator. After the incubation suspension was cooled with water at 20°C to 25°C, 600 µL of TBS was added to obtain a suspension of nucleic acid-encapsulating nanoparticles (nucleic acid concentration: 5 µg/mL).

The particle size, polydispersity index (PdI), and mRNA encapsulation rate of the obtained nucleic acid-encapsulating nanoparticles of Examples 6 to 9 were analyzed on each time point of Day 0, Day 7, and Day 14. The particle size and PdI were measured by a dynamic light scattering method using Zetasizer (registered trademark). The mRNA encapsulation rate was measured by Ribogreen (registered trademark) assay. As a result, no change was observed in the particle properties during storage for 2 weeks, and the particle properties were maintained, indicating high storage stability (see Table 6).

**[Table 6]**

| | time point | encapsulation rate (%) | Z-Ave (d.nm) | Number Mean (d.nm) | PdI |
|---|---|---|---|---|---|
| Example 6 | Day 0 | 99 | 169 | 126 | 0.12 |
| Example 7 | | 98 | 159 | 119 | 0.12 |
| Example 8 | | 84 | 222 | 154 | 0.16 |
| Example 9 | | 80 | 214 | 159 | 0.16 |
| Example 6 | Day 7 | 97 | 180 | 127 | 0.15 |
| Example 7 | | 96 | 154 | 118 | 0.10 |
| Example 8 | | 80 | 237 | 191 | 0.15 |
| Example 9 | | 72 | 227 | 162 | 0.19 |
| Example 6 | Day 14 | 96 | 168 | 124 | 0.11 |
| Example 7 | | 94 | 164 | 120 | 0.11 |
| Example 8 | | 77 | 236 | 185 | 0.16 |
| Example 9 | | 64 | 274 | 155 | 0.19 |

The nucleic acid-encapsulating nanoparticles obtained were applied to HeLa cells to evaluate the nucleic acid transfer efficiency. HeLa cells were seeded in D-MEM medium (high glucose) (containing L-glutamine and phenol red) (containing 10% FBS by volume and 1% penicillin-streptomycin by volume) at 3×10⁴ cells/600 µL into a 24-well flat-bottom transparent black plate. The obtained suspension of nucleic acid-encapsulated nanoparticles was added such that the nucleic acid concentration in the medium was 0.12 µg/60 µL. Luciferin was added to the medium to 0.1 mM, and the amount of luciferase protein introduced was evaluated over time using the amount of luminescence. HeLa cells cultured in a medium containing nucleic acid-encapsulating nanoparticles and luciferin were placed in an incubator-type luminometer (Kronos), and the cumulative amount of luminescence was measured every hour. As a result, the transfer rate was maintained in all formulations even after 14 days of storage, demonstrating high storage stability (see Fig. 5). In Fig. 5, "1.8.E+08", etc. mean "1.8×10⁸" and the like.

### [Industrial Applicability]

According to the present invention, since nucleic acid can be intracellularly transferred with high efficiency, the present invention is useful for nucleic acid medicaments, gene therapy and biochemical experiments.

This application is based on patent application No. 2022-114394 filed in Japan, the contents of which are encompassed in full herein.

## Claims

1. A lyophilized composition of nucleic acid-encapsulating lipid nanoparticles having a pH of 4.5 or more and 6.9 or less, comprising an ionic lipid represented by the formula (1): 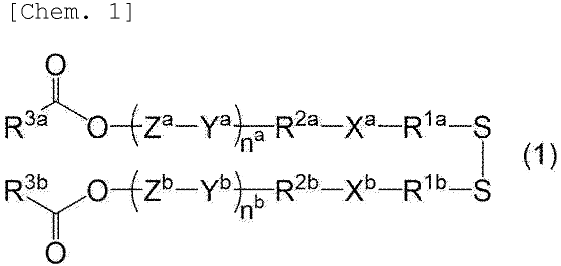 (in the formula (1),
R^{1a} and R^{1b} are each independently an alkylene group having 1 to 6 carbon atoms,
X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
R^{2a} and R^{2b} are each independently an alkylene group or an oxydialkylene group each having 8 or less carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 to 16 carbon atoms and at least one aromatic ring, and optionally having a hetero atom,
n^{a} and n^{b} are each independently 0 or 1, and
R^{3a} and R^{3b} are each independently
a residue derived from a reaction product of a liposoluble vitamin having a hydroxyl group, and succinic anhydride or glutaric anhydride,
a residue derived from a reaction product of a sterol derivative having a hydroxyl group, and succinic anhydride or glutaric anhydride,
an aliphatic hydrocarbon group having 1 to 40 carbon atoms,
an alkyl group having 3 to 40 carbon atoms and a cyclopropane ring,
a group represented by the formula (3):
R⁹-O-CO-(CH₂)ₐ-* (3)
(in the formula (3),
* is a bonding position,
R⁹ is an aliphatic hydrocarbon group having 2 to 20 carbon atoms, and
a is an integer of 2 to 10),
a group having 50 or less carbon atoms and represented by the formula (4): 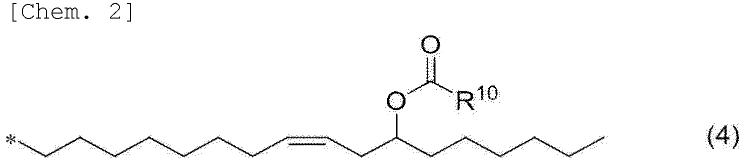 (in the formula (4),
* is a bonding position, and
R¹⁰ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and R¹⁰ is optionally substituted by a substituent selected from the group consisting of a 3- to 14-membered heterocyclic group and a hydrocarbon ring group having 3 to 12 carbon atoms), or
a group having 50 or less carbon atoms and represented by the formula (5): 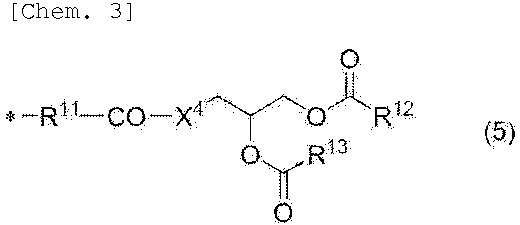 (in the formula (5),
* is a bonding position,
R¹¹ is an alkylene group having 2 to 9 carbon atoms, an alkenediyl group having 2 to 9 carbon atoms, or an alkynediyl group having 2 to 9 carbon atoms, and R¹¹ is optionally substituted by a substituent selected from the group consisting of a halogen atom, a hydroxyl group, and a hydrocarbon ring group having 3 to 12 carbon atoms;
R¹² and R¹³ are each independently an alkyl group having 1 to 17 carbon atoms, an alkenyl group having 2 to 17 carbon atoms, or an alkynyl group having 2 to 17 carbon atoms, and at least one ethylene group or at least one trimethylene group in R¹² is optionally replaced with at least one bond selected from the group consisting of an ester bond, an amide bond, a carbamate bond, and a carbonate bond, at least one ethylene group or at least one trimethylene group in R¹³ is optionally replaced with at least one bond selected from the group consisting of an ester bond, an amide bond, a carbamate bond, and a carbonate bond, and R¹² and R¹³ are each independently optionally substituted by a substituent selected from the group consisting of a halogen atom, a hydroxyl group, and a hydrocarbon ring group having 3 to 12 carbon atoms, and
X⁴ is an oxygen atom, NH, or a sulfur atom).

2. The lyophilized composition according to claim 1, wherein
R^{3a} and R^{3b} are each independently
a residue derived from a reaction product of a liposoluble vitamin having a hydroxyl group, and succinic anhydride or glutaric anhydride,
a residue derived from a reaction product of a sterol derivative having a hydroxyl group, and succinic anhydride or glutaric anhydride,
an aliphatic hydrocarbon group having 1 to 40 carbon atoms,
an alkyl group having 3 to 40 carbon atoms and a cyclopropane ring, or
a group represented by the formula (3):
R⁹-O-CO-(CH₂)ₐ-* (3)
(the symbols in the formula (3) are defined as in [1]).

3. The lyophilized composition according to claim 1, wherein the ionic lipid is a compound represented by the following formula:

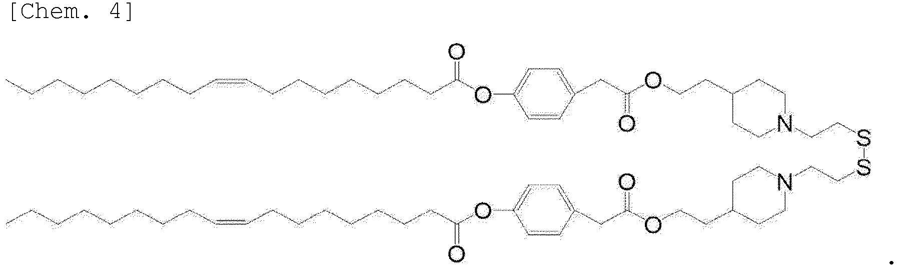

4. The lyophilized composition according to claim 3, further comprising a compound represented by the following formula: 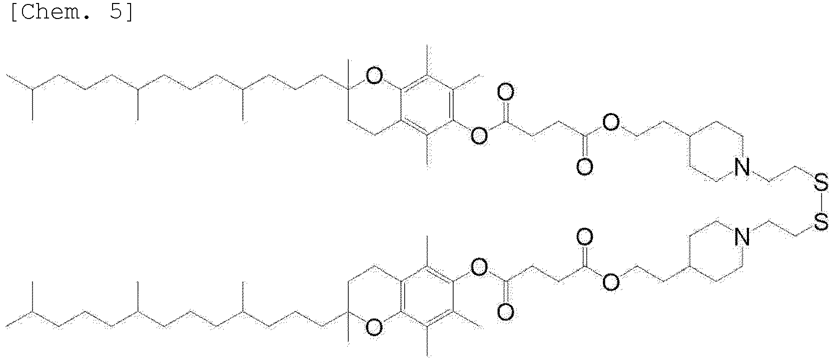 as the ionic lipid.

5. The lyophilized composition according to claim 1, wherein the ionic lipid is a compound represented by the following formula:

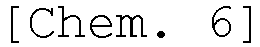

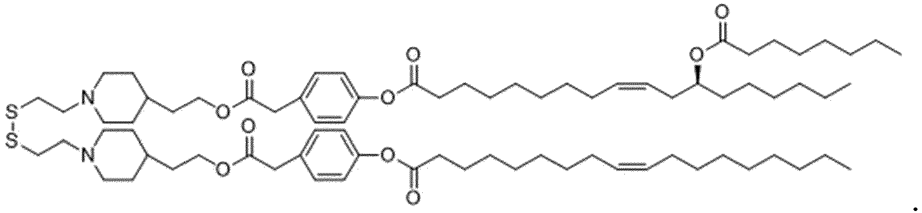

6. The lyophilized composition according to claim 1, wherein the ionic lipid is a compound represented by the following formula:

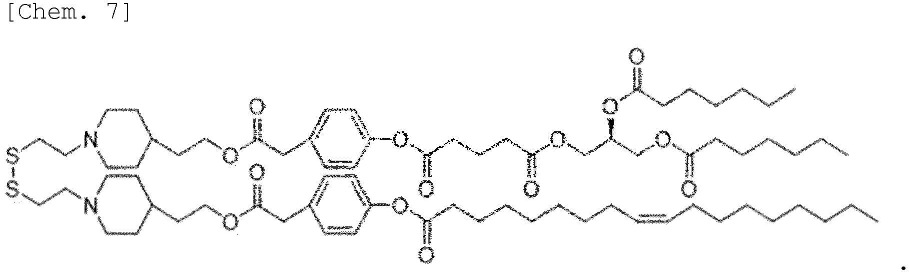

7. The lyophilized composition according to any one of claims 1 to 6, wherein the nucleic acid encapsulated in the lipid nanoparticles is mRNA.

8. A method for producing a lyophilized composition of nucleic acid-encapsulating lipid nanoparticles, comprising the following steps:
a) mixing an alcohol solution comprising an ionic lipid represented by the formula (1) in claim 1, a sterol, a phospholipid, and a PEG lipid with a nucleic acid solution in an acidic buffer having a buffering action at pH 1.0 to 6.5 to prepare a suspension of nucleic acid-encapsulating lipid nanoparticles;
b) exchanging an external aqueous phase of the nucleic acid-encapsulating lipid nanoparticles with another buffer having a buffering action at pH 4.5 to 6.9 to obtain a mixture comprising the nucleic acid-encapsulating lipid nanoparticles at pH 4.5 to 6.9;
c) mixing the obtained mixture with a cryoprotectant to obtain a mixture comprising 80 to 320 mg/mL of the cryoprotectant and the nucleic acid-encapsulating lipid nanoparticles at pH 4.5 to 6.9;
d) lyophilizing the mixture obtained in step c) to obtain the lyophilized composition.

9. A method for producing a nucleic acid-encapsulating lipid nanoparticle, comprising the following steps:
a) mixing an alcohol solution comprising an ionic lipid represented by the formula (1) in claim 1, a sterol, a phospholipid, and a PEG lipid with a nucleic acid solution in an acidic buffer having a buffering action at pH 1.0 to 6.5 to prepare a suspension of nucleic acid-encapsulating lipid nanoparticles;
b) exchanging an external aqueous phase of the nucleic acid-encapsulating lipid nanoparticles with another buffer having a buffering action at pH 4.5 to 6.9 to obtain a mixture comprising the nucleic acid-encapsulating lipid nanoparticles at pH 4.5 to 6.9;
c) mixing the obtained mixture with a cryoprotectant to obtain a mixture comprising 80 to 320 mg/mL of the cryoprotectant and the nucleic acid-encapsulating lipid nanoparticles at pH 4.5 to 6.9;
d) lyophilizing the mixture obtained in step c) to obtain a lyophilized composition;
e) mixing the lyophilized composition with water and optionally incubating the mixture at 0 to 95°C for 0 to 60 minutes to obtain the nucleic acid-encapsulating lipid nanoparticles.

10. A method for transferring nucleic acid into cells, comprising a step of contacting the nucleic acid-encapsulating lipid nanoparticle produced by the method according to claim 9 with cells in vitro.

11. A method for transferring nucleic acid into target cells, comprising a step of administering the nucleic acid-encapsulating lipid nanoparticle produced by the method according to claim 9 to a living body.

12. A method for producing a pharmaceutical composition, comprising the method according to claim 8.

13. A method for producing a pharmaceutical composition, comprising the method according to claim 9.
